# EUROPÄISCHE PATENTSCHRIFT

(11) **EP 0 946 492 B1**
(45) Veröffentlichungstag und Bekanntmachung des Hinweises auf die Patenterteilung: **18.06.2003**
(21) Anmeldenummer: 97953757.8
(22) Anmeldetag: 08.12.1997
(51) Int. Cl.: C07C 69/653, C07D 213/61, C07D 213/26, A01N 43/40, A01N 31/02

(54) **NEUE 2-FLUORACRYLSÄUREDERIVATE, NEUE MISCHUNGEN AUS HERBIZIDEN UND ANTIDOTS UND DEREN VERWENDUNG**
NOVEL 2-FLUOROACRYLIC ACID DERIVATIVES, NOVEL MIXTURES OF HERBICIDES AND ANTIDOTES AND THE USE THEREOF
NOUVEAUX DERIVES D'ACIDE 2-FLUOROACRYLIQUE, NOUVEAUX MELANGES D'HERBICIDES ET D'ANTIDOTES ET LEUR UTILISATION

(30) Priorität: 19.12.1996 DE 19652961
(43) Veröffentlichungstag der Anmeldung: 06.10.1999
(73) Patentinhaber: Bayer CropScience GmbH, 65929 Frankfurt/Main (DE)
(72) Erfinder: ZIEMER, Frank, D-65830 Kriftel (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE); DEMASSEY, Jacques, F-77144 Chalifert (FR)
(86) Internationale Anmeldenummer: EP9706846
(87) Internationale Veröffentlichungsnummer: WO98027049

(56) Entgegenhaltungen:
- EP-A- 0 012 158
- EP-A- 0 547 972
- WO-A-91/16301
- PIRRUNG, MICHAEL C. ET AL: "Mechanistic and stereochemical study of phenylpyruvate tautomerase" J. AM. CHEM. SOC. (1993), 115(16), 7103-10 CODEN: JACSAT;ISSN: 0002-7863, XP002064658
- E.D. BERGMAN ET AL.: "Organic fluorine compounds" JOURNAL OF THE CHEMICAL SOCIETY, SECTION C: ORGANIC CHEMISTRY., Nr. 10, 1968, LETCHWORTH GB, Seiten 1232-1235, XP002064659
- R. ARNAUD ET AL.: "The relationship between fluorine-19 substituent chemical shifts and electron populations in para and meta substituted alpha-fluoro-Z-cinnamic esters" JOURNAL OF FLUORINE CHEMISTRY., Bd. 48, Nr. 2, 1990, LAUSANNE CH, Seiten 281-292, XP002064660
- J.F. NORMANT ET AL.: "New preparation of trifluorovinyllithium" SYNTHESIS., Nr. 2, 1975, STUTTGART DE, Seiten 122-125, XP002064661
- E. ELKIK ET AL.: "Preparation and proton, fluorine, and carbon-13 NMR spectra of m- and p-substituted alpha-fluorocinnamic esters" BULLETIN DE LA SOCIETE CHIMIQUE DE FRANCE., Nr. 3, 1986, PARIS FR, Seiten 423-428, XP002064662
- ROBINSON C N ET AL: "13C AND 19F NMR SPECTRA OF SUBSTITUTED ETHYL ALPHA -FLUOROCINNAMATES. APPLICATION OF THE DSP-NLR EQUATION" TETRAHEDRON, Bd. 46, Nr. 2, 1990, Seiten 335-340, XP000673161
- E. ELKIK ET AL.: "Reaction of dimethylsulfoxonium methylide with alpha-fluoro-alpha-ethylenic esters" BULLETIN DE LA SOCIETE CHIMIQUE DE FRANCE., Nr. 5, 1987, PARIS FR, Seiten 861-866, XP002064663
- CHEMICAL ABSTRACTS, vol. 124, no. 5, 29.Januar 1996 Columbus, Ohio, US; abstract no. 55063g, H.-J. TSAI ET AL.: "A one-pot synthesis of unsymmetrical and symmetrical tetrasubstituted alpha-fluoro-alpha,beta-unsaturated esters." Seite 1148; XP002064664 & PHOSPHORUS, SULFUR AND SILICON AND THE RELATED ELEMENTS; ISSN 1042-6507, Bd. 105, Nr. 1-4, 1995, LONDON, Seiten 205-212,

## Beschreibung

Die Erfindung betrifft das technische Gebiet der Pflanzenschutzmittel, insbesondere α-Fluoracrylsäurederivate sowie Wirkstoff-Antidot-Kombinationen, die hervorragend für den Einsatz gegen konkurrierende Schadpflanzen in Nutzpflanzenkulturen geeignet sind.

Bei der Anwendung von Pflanzenbehandlungsmitteln, insbesondere bei der Anwendung von Herbiziden, können unerwünschte Schäden an den behandelten Kulturpflanzen auftreten. Insbesondere dann, wenn die Herbizide gegenüber wichtigen Kulturpflanzen nicht voll verträgtich (nicht selektiv) sind, sind ihrem Einsatz enge Grenzen gesetzt. Sie können daher manchmal nicht oder nur in so geringen Aufwandmengen eingesetzt werden, daß die erwünschte breite herbizide Wirksamkeit nicht gewährleistet ist. So können z. B. viele Herbizide aus der Reihe der Sulfonylhamstoffe nicht selektiv in Mais eingesetzt werden. Besonders bei der Nachauflaufapplikation von Herbiziden ist es wünschenswert, eine derartige Phytotoxizität zu verringern.

Aus DE OS 1 542 872 sind α-Chlor-Zimtsäurederivate als herbizide Mittel bekannt. Weiterhin sind in NL 7 102 436 Herstellungsverfahren von Arylalkancarbonsäuren als Pflanzenwachstumsregulatoren beschrieben.

Von Bergmann und Shahak, J. Chem. Soc., 1961, 4033-4038 sind bereits α-Fluor-Zimtsäurederivate sowie α-Fluor-Pyridylacrylsäurederivate beschrieben worden. Weitere 3- oder 4-substituierte α-Fluor-Zimtsäurederivate wurden von Robinson und Stablein, (1990), Tetrahedron 46(2), 335-340 beschrieben.

Weiterhin sind einige Derivate von 3- oder 4-substituierten und 3,4-disubstituierten α-Fluor-Zimtsäuren aus J. Am. Chem. Soc. 1993, Vol. 115, 7103-7110; J. Chem. Soc. 1968, 1232-1235; J. Fluorie Chem. 1990, Vol. 48, 281-292; Bull. Soc. Chim. Fr. 1986, 423-428; Bull. Soc. Chim. Fr. 1987, 861-866; WO 91/16301 und EP-A 0 547 972 bekannt Aus Synthesis 1975, 122-125 und Phosph., Sulfur and Silicon, 1995, Vol. 105, 205-212 sind darüber hinaus einige Derivate der α-Fluor-β-methylzimtsäure bekannt. Die vorstehend genannten Schriften beschreiben diese Verbindungen im Zusammenhang mit mechanistischen, stereochemischen oder spektroskopischen Studien. Diese Schriften offenbaren dabei nicht die Safener-Eigenschaften solcher Verbindungen.

Ganz unerwartet haben neue experimentelle Arbeiten gezeigt, daß α-Fluoracrylsäurederivate hervorragend dazu geeignet sind, die phytotoxischen Nebenwirkungen von Herbiziden an Kulturpflanzen deutlich zu vermindern oder vollständig aufzuheben.

Gegenstand der vorliegenden Erfindung sind daher Herbizid-Safener-Kombinationen enthaltend
A) mindestens einen herbiziden Wirkstoff sowie
B) mindestens ein 2-Fluoracrylsäurederivat der Formel (I), 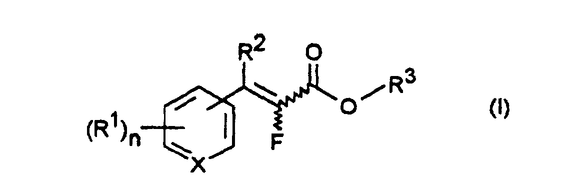
in welcher
- X: CH oder N bedeutet;
- n: für den Fall, daß X = N ist, eine ganze Zahl von 0 bis 4 und für den Fall, daß X = CH ist, eine ganze Zahl von 0 bis 5 bedeutet;
- R¹: Halogen, (C₁-C₄)-Alkyl, (C₁-C₄)-Halogenalkyl, (C₁-C₄)-Alkoxy, (C₁-C₄)-Halogenalkoxy, Nitro, (C₁-C₄)-Alkylthio, (C₁-C₄)-Alkylsulfonyl, (C₁-C₄)-Alkoxycarbonyl oder Phenyl oder Phenoxy bedeutet, wobei die beiden letztgenannten Reste unsubstituiert oder durch einen oder mehrere, vorzugsweise bis zu drei gleiche oder verschiedene Reste aus der Gruppe, bestehend aus Halogen, (C₁-C₄)-Alkyl, (C₁-C₄)-Halogenalkyl, (C₁-C₈)-Alkoxy, Halogen-(C₁-C₈)-alkoxy, Nitro, Amino, Mono- oder Di-(C₁-C₄)-alkylamino und Cyano,substituiert ist, oder auch für den Fall, daß n eine ganze Zahl größer 1 ist, zwei der Reste R¹ gemeinsam ein unsubstituiertes oder substituiertes 1,ω-Dioxoalkylen bedeuten;
- R²: Wasserstoff oder (C₁-C₄)-Alkyl bedeutet und
- R³: Wasserstoff, (C₁-C₈)-Alkyl,(C₂-C₄)-Alkenyl oder(C₂-C₄)-Alkinyl bedeutet, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und (C₁-C₄)-Alkoxy substituiert ist,
oder deren Salze.

In Formel (I) und allen nachfolgenden Formeln können die Reste Alkyl, Alkoxy, Halogenalkyl, Halogenalkoxy, Alkylamino und Alkylthio sowie die entsprechenden ungesättigten und/oder substituierten Reste im Kohlenstoffgerüst jeweils geradkettig oder verzweigt sein.

Alkylreste, auch in den zusammengesetzten Bedeutungen wie Alkoxy, Halogenalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet Fluor, Chlor, Brom oder lod, vorzugsweise Fluor oder Chlor; Halogenalkyl bedeutet durch Halogen, vorzugsweise durch Fluor, Chlor und/oder Brom, insbesondere durch Fluor oder Chlor, teilweise oder vollständig substituiertes Alkyl wie Monohalogenalkyl, Perhalogenalkyl, CF₃, CHF₂, CH₂F, CF₃CF₂, CH₂FCHCl, CCl₃, CHCl₂, CH₂CH₂Cl; Halogenalkoxy ist z.B. OCF₃, OCHF₂, OCH₂F, OCF₂CF₃, OCH₂CF₃ und OCH₂CH₂Cl

Substituiertes Dioxoalkylen ist z.B. ein Dioxoalkylenrest, der ein- oder mehrfach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, vorzugsweise ein 1,ω-Dioxoalkylenrest.

Die Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die vorliegende Erfindung betrifft die Verbindungen der Formel (I) in Form der freien Base oder eines Salzes, vorzugsweise eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, ölsäure, Methansulfonsäure, Benzolsulfonsäure oder Toluolsulfonsäure. Weitere, besonders bevorzugte Salze sind möglich, wenn saure Gruppen wie Carboxy oder phenolisches Hydroxy enthalten sind, bei denen ein Wasserstoff durch ein für die Landwirtschaft geeignetes Kation ersetzt wird. Salze der Verbindungen der Formel (I) sind beispielsweise Metallsalze, insbesondere Alkalimetallsalze oder Erdalkalimetallsalze, insbesondere Natrium-, Kalium- und Ammoniumsalze oder Salze mit organischen Aminen wie z. B. (C₁-C₄)-Alkylamine oder (C₁-C₄)-Hydroxyalkylamine. Außerdem können durch Umsetzung von basischen Gruppen wie gegebenenfalls substituierten Aminogruppen oder basischen Heterocyclen mit anorganischen oder organischen Säuren Säureadditionssalze gebildet werden.

Die Erfindung betrifft auch ein Verfahren zum Schutz von Kulturpflanzen, vorzugsweise Getreidekulturen (Weizen, Roggen, Gerste, Hafer, Reis, Mais, Sorghum), aber auch Baumwolle und Sojabohne, insbesondere Getreide, besonders bevorzugt Maispflanzen, vor phytotoxischen Nebenwirkungen von Herbiziden, insbesondere Sulfonylharnstoffherbiziden, dadurch gekennzeichnet, daß eine wirksame Menge mindestens einer Verbindung der Formel (I) vor, nach oder gleichzeitig mit dem obengenannten herbiziden Wirkstoff auf die Pflanzen, Pflanzensamen oder die Anbaufläche appliziert wird.

Die Erfindung betrifft weiterhin die Verwendung von Verbindungen der Formel (I) zum Schutz von Kulturpflanzen, vorzugsweise Getreide- oder Maispflanzen, vor phytotoxischen Nebenwirkungen von Herbiziden, insbesondere Sulfonylharnstoffherbiziden.

Die Erfindung betrifft außerdem neue 2-Fluoracrylsäurederivate der Formel (I) 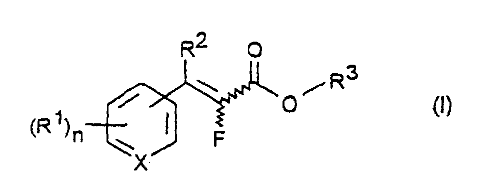 in welcher
X, n, R¹, R² und R³ wie oben definiert sind
oder deren Salze,
ohne dabei eine der nachfolgenden Verbindungen zu bedeuten:
a) α-Fluorzimtsäure oder deren Methyl- oder Ethylester,
b) 3- oder 4-Methyl-α-fluorzimtsäureethylester,
c) 3- oder 4-Chlor-α-fluorzimtsäure oder deren Methyl- oder Ethylester,
d) 2-Hydroxy-α-fluorzimtsäureethylester,
e) α-Fluor-α-2-pyridylacrylsäureethylester,
f) α-Fluor-α-3-pyridylacrylsäureethylester,
g) 3- oder 4-Methoxy-α-fluorzimtsäureethylester,
h) 3- oder 4-Phenoxy-α-fluorzimtsäureethylester,
j) 4-Phenyl-α-fluorzimtsäureethylester,
k) 3- oder 4-Fluor-α-fluorzimtsäure oder deren Methyl- oder Ethylester,
l) 3- oder 4-Brom-α-fluorzimtsäure oder deren Ethylester,
m) 4-Carboxyethyl-α-fluorzimtsäureethylester,
n) 3- oder 4-Trifluormethyl-α-fluorzimtsäuremethyl- oder ethylester,
o) 3- oder 4-Cyano-α-fluorzimtsäureethylester,
p) 3- oder 4-Nitro-α-fluorzimtsäureethylester,
q) 3,4-Dichlor-α-fluorzimtsäureethylester,
r) α-Fluor-β-methylzimtsäure oder deren Ethylester,
s) 4-Methyl-α-fluorzimtsäure-t-butylester,
t) 3,4-Dibrom-α-fluorzimtsäureethylester,
u) β-Ethyl-α-fluor-zimtsäureethylester zu bedeuten.

Die Verbindungen der allgemeinen Formel (I) lassen sich nach allgemein bekannten Verfahren herstellen [Robinson et al., Tetrahedron 46 (1990) 335-340; Bergman et al., J. Chem. Soc., (1961), 4033-4038; Ishihara et al., Chem. Lett., (1987), 1145-1148; US 4,338,253; Piva, Synlett, (1994), 729-731; Bergmann et al., J. Chem. Soc., (1968), 1232-1235].

Die Erfindung betrifft darüber hinaus ein Verfahren zur Herstellung von 2-Fluoracrylsäurederivaten der Formel (I), worin man einen Aldehyd oder Keton der Formel (II). 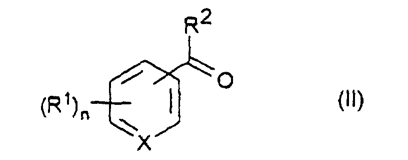 worin
X, R¹, R² und n wie in Formel (I) definiert sind, mit 2-Fluor-2-phosphonoessigsäuretriethylester zum Ethylester umsetzt und diesen, falls R³ ungleich Ethyl ist, anschließend nach üblichen Verfahren zur Verbindung der Formel (I) umsetzt.

Die Herstellung der überwiegend Z-konfigurierten Verbindungen der Formel (I_{Z}) kann z. B. in der Weise erfolgen, daß man gemäß nachfolgendem Schema einen Aldehyd oder ein Keton der Formel (II), 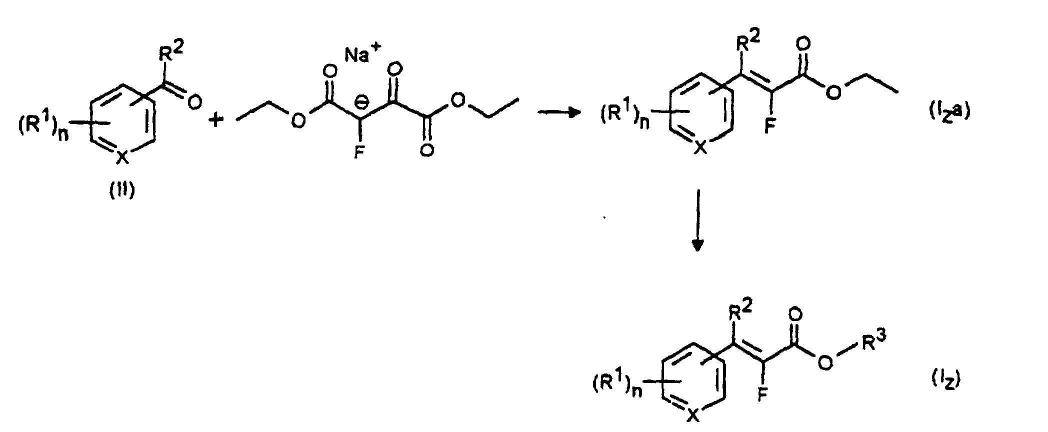 worin
X, R¹, R² und n wie in Formel (I) definiert sind, mit dem aus Fluoressigsäureethylester und Oxalsäurediethylester unter Verwendung von Natriumhydrid zugänglichen Natriumsalz des Diethyloxalofluoroacetats zunächst zum Ethylester (I_{Z}a) umsetzt, um diesen anschließend nach üblichen Verfahren umzuestem (Variante 1).

Die E-konfigurierten Verbindungen der Formel (I_{E}) sind beispielsweise zugänglich, indem man gemäß nachfolgendem Schema einen Aldehyd oder ein Keton der Formel (II), 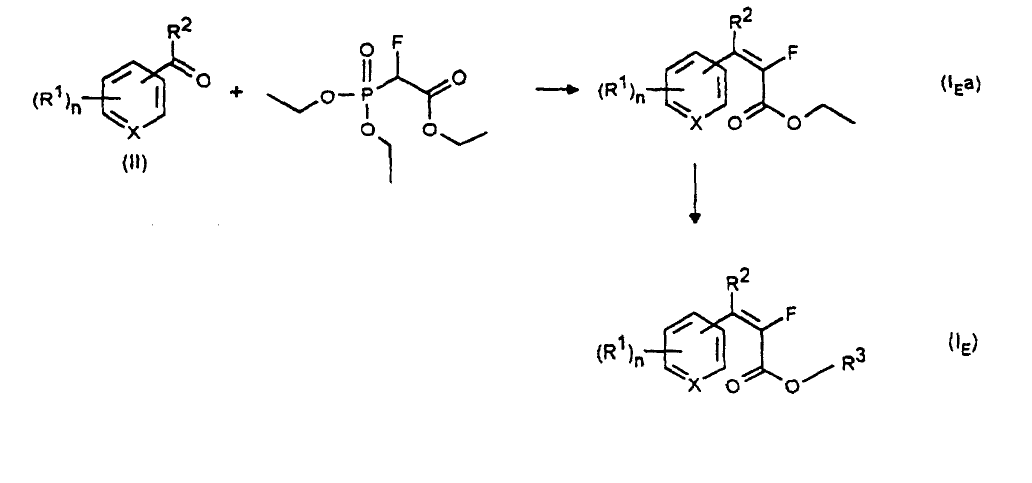 worin
X, R¹, R² und n wie in Formel (I) definiert sind, mit 2-Fluor-2-phosphonoessigsäuretriethylester in Gegenwart von Butyllithium zunächst zum Ethylester (I_{E}a) umsetzt, um diesen anschließend nach üblichen Verfahren umzuestem (Variante 2).

Die Umsetzungen nach Variante 1 erfolgen vorzugsweise in einem inerten organischen Lösungsmittel oder Lösungsmittelgemisch. Als Lösungsmittel eignen sich beispielsweise Tetrahydrofuran (THF), Dioxan, Acetonitril oder Dimethylformamid.

Die Reaktionstemperaturen liegen vorzugsweise im Bereich zwischen -20°C und 100°C.

Die Umsetzungen nach Variante 2 erfolgen ebenfalls vorzugsweise in einem inerten organischen Lösungsmittel oder Lösungsmittelgemisch in Gegenwart mindestens einer starken Base wie z. B. Butyllithium. Als Lösungsmittel eignet sich beispielsweise THF.

Die Reaktionstemperaturen liegen vorzugsweise im Bereich zwischen -100°C und 20°C.

Werden die erfindungsgemäßen Safener der Formel (I) in subtoxischen Konzentrationen zusammen mit den herbiziden Wirkstoffen oder auch in einer beliebigen Reihenfolge ausgebracht, so sind sie in der Lage, die phytotoxischen Nebenwirkungen dieser Herbizide zu reduzieren bzw. völlig aufzuheben, ohne jedoch die Wirksamkeit der Herbizide gegenüber den Schadpflanzen zu vermindern.

Geeignete Herbizide, die mit den erfindungsgemäßen Safenem kombiniert werden können, sind beispielsweise:
A) Herbizide vom Typ der Phenoxyphenoxy- und Heteroaryloxyphenoxycarbonsäure-(C₁-C₄)-alkyl-, (C₂-C₄)-alkenyl- und (C₃-C₄)-alkinylester wie
   A1) Phenoxy-phenoxy- und Benzyloxy-phenoxy-carbonsäure-derivate, z.B.
      2-(4-(2,4-Dichlorphenoxy)-phenoxy)-propionsäuremethylester (Diclofopmethyl),
      2-(4-(4-Brom-2-chlorphenoxy)-phenoxy)-propionsäuremethylester (s. DE-A-2601548),
      2-(4-(4-Brom-2-fluorphenoxy)-phenoxy)-propionsäuremethylester (s. US-A-4808750),
      2-(4-(2-Chlor-4-trifluormethylphenoxy)-phenoxy)-propionsäuremethylester (s. DE-A-2433067),
      2-(4-(2-Fluor-4-trifluormethylphenoxy)-phenoxy)-propionsäuremethylester (s. US-A-4808750),
      2-(4-(2,4-Dichlorbenzyl)-phenoxy)propionsäuremethylester (s. DE-A-2417487),
      4-(4-(4-Trifluormethy(phenoxy)-phenoxy)-pent-2-en-säureethylester,
      2-(4-(4-Trifluormethylphenoxy)-phenoxy)-propionsäuremethylester (s. DE-A-2433067),
   A2) "Einkernige" Heteroaryloxy-phenoxy-alkancarbonsäurederivate, z.B.
      2-(4-(3,5-Dichlorpyridyl-2-oxy)-phenoxy)-propionsäureethylester (s. EP-A-2925),
      2-(4-(3,5-Dichlorpyridyl-2-oxy)-phenoxy)-propionsäurepropargylester (s. EP-A-3114),
      2-(4-(3-Chlor-5-trifluormethyl-2-pyridyloxy)-phenoxy-propionsäure-methylester (s. EP-A-3890),
      2-(4-(3-Chlor-5-trifluormethyl-2-pyridyloxy)-phenoxy)-propionsäure-ethylester (s. EP-A-3890),
      2-(4-(5-Chlor-3-fluor-2-pyridyloxy)-phenoxy)-propionsäurepropargylester (EP-A-191736),
      2-(4-(5-Trifluormethyl-2-pyridyloxy)-phenoxy)-propionsäurebutylester (Fluazifopbutyl),
   A3) "Zweikernige" Heteroaryloxy-phenoxy-alkancarbonsäurederivate, z.B.
      2-(4-(6-Chlor-2-chinoxalyloxy)-phenoxy)-propionsäuremethylester und -ethylester (Quizalofop-methyl und -ethyl),
      2-(4-(6-Fluor-2-chinoxalyloxy)-phenoxy)-propionsäuremethylester (s. J. Pest. Sci. Vol. 10, 61 (1985)),
      2-(4-(6-Chlor-2-chinoxalyloxy)-phenoxy)-propionsäure und -2-isopropylidenaminooxyethylester (Propaquizafop u. Ester),
      2-(4-(6-Chlorbenzoxazol-2-yl-oxy)-phenoxy)-propionsäureethylester (Fenoxaprop-ethyl), dessen D(+) Isomer (Fenoxaprop-P-ethyl) und
      2-(4-(6-Chlorbenzthiazol-2-yloxy)-phenoxypropionsäureethylester (s. DE-A-2640730),
      2-(4-(6-Chlorchinoxalyloxy)-phenoxy-propionsäure-tetrahydrofur-2-ylmethyl-ester (s. EP-A 323 727),
B) Herbizide aus der Sulfonylhamstoff-Reihe, wie z. B. Pyrimidin- oder Triazinylaminocarbonyl-[benzol-, pyridin-, pyrazol-, thiophen- und (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig von-einander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Halogenalkoxy, Halogenalkyl, Alkylcarbonyl, Alkoxyalkyl, (Alkansulfonyl)alkylamino. Geeignete Sulfonylhamstoffe sind beispielsweise
   B1) Phenyl- und Benzylsulfonylharnstoffe und verwandte Verbindungen, z. B.
      1-(2-Chlorphenylsulfonyl)-3-(4-methoxy-6-methyl-1,3,5-triazin-2-yl)harnstoff (Chlorsulfuron),
      1-(2-Ethoxycarbonylphenylsulfonyl)-3-(4-chlor-6-methoxypyrimidin-2-yl)-harnstoff (Chlorimuron-ethyl),
      1-(2-Methoxyphenylsulfonyl)-3-(4-methoxy-6-methyl-1,3,5-triazin-2-yl)-hamstoff (Metsulfuron-methyl),
      1-(2-Chlorethoxyphenylsulfonyl)-3-(4-methoxy-6-methyl-1,3,5-triazin-2-yl)-harnstoff (Triasulfuron),
      1-(2-Methoxycarbonylphenylsulfonyl)-3-(4,6-dimethylpyrimidin-2-yl)harnstoff (Sulfometuron-methyl),
      1-(2-Methoxycarbonylphenylsulfonyl)-3-(4-methoxy-6-methyl-1,3,5-triazin-2-yl)-3-methylhamstoff (Tribenuron-methyl),
      1-(2-Methoxycarbonylbenzylsulfonyl)-3-(4,6-dimethoxylpyrimidin-2-yl)-harnstoff (Bensulfuron-methyl),
      1-(2-Methoxycarbonylphenylsulfonyl)-3-(4,6-bis-(difluormethoxy)pyrimidin-2-yl)hamstoff (Primisulfuron-methyl),
      3-(4-Ethyl-6-methoxy-1,3,5-triazin-2-yl)-1-(2,3-dihydro-1,1-dioxo-2-methylbenzo[b]thiophen-7-sulfonyl)harnstoff (s. EP-A-79683),
      3-(4-Ethoxy-6-ethyl-1,3,5-triazin-2-yl)-1-(2,3-dihydro-1,1-dioxo-2-methylbenzo[b]thiophen-7-sulfonyl)harnstoff (s. EP-A-79683),
      3-(4-Methoxy-6-methyl-1,3,5-triazin-2-yl)-1-(2-methoxycarbonyl-5-iodphenylsulfonyl)-harnstoff (s. WO 92/13845),
      DPX-66037, Triflusulfuron-methyl (s. Brighton Crop Prot. Conf. - Weeds - 1995, S. 853),
      CGA-277476, (s. Brighton Crop Prot. Conf. - Weeds - 1995, S. 79), Methyl-2-[3-(4,6-dimethoxypyrimidin-2-yl)ureidosulfonyl]-4-methansulfonamidomethyl-benzoat (s. WO 95/10507),
      N,N-Dimethyl-2-[3-(4,6-dimethoxypyrimidin-2-yl)ureidosulfonyl]-4-formylaminobenzamid (s. PCTIEP 95/01344),
   B2) Thienylsulfonylharnstoffe, z. B.
      1-(2-Methoxycarbonylthiophen-3-yl)-3-(4-methoxy-6-methyl-1,3,5-triazin-2-yl)hamstoff (Thifensulfuron-methyl),
   B3) Pyrazolylsulfonylharnstoffe, z. B.
      1-(4-Ethoxycarbonyl-1-methylpyrazol-5-yl-sulfonyl)-3-(4,6-dimethoxypyrimidin-2-yl)hamstoff (Pyrazosulfuron-methyl)
      Methyl-3-chlor-5-(4,6-dimethoxypyrimidin-2-ylcarbamoylsulfamoyl)-1-methylpyrazol-4-carboxylat (s. EP 282613)
      5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference - Weeds - 1991, Vol. 1, S. 45 ff.),
      DPX-A8947, Azimsulfuron, (s. Brighton Crop Prot. Conf. - Weeds- 1995, S 65),
   B4) Sulfondiamid-Derivate, z.B.
      3-(4,6-Dimethoxypyrimidin-2-yl)-1-(N-methyl-N-methylsulfonylaminosulfonyl)-harnstoff (Amidosulfuron) und Strukturanaloge (s. EP-A-131258 und Z. Pfl. Krankh. Pfl. Schutz, Sonderheft XII, 489-497 (1990)),
   B5) Pyridylsulfonylharnstoffe, z.B.
      1-(3-N,N-Dimethylaminocarbonylpyridin-2-ylsulfonyl)-3-(4,6-dimethoxypyrimidin-2-yl)hamstoff (Nicosulfuron),
      1-(3-Ethylsulfonylpyridin-2-ylsulfonyl)-3-(-(4,6-dimethoxypyrimidin-2-yl)-hamstoff (Rimsulfuron),
      2-[3-(4,6-Dimethoxypyrimidin-2-yl)ureidosulfonyl]-6-trifluormethyl-3-pyridincarbonsäuremethylester, Natriumsalz (DPX-KE459, Flupyrsulfuron, s. Brighton Crop Prot. Conf. - Weeds - 1995, S. 49),
      Pyridylsulfonylharnstoffe, wie sie in DE-A-4000503 und DE-A-4030577 beschrieben sind, vorzugsweise solche der Formel 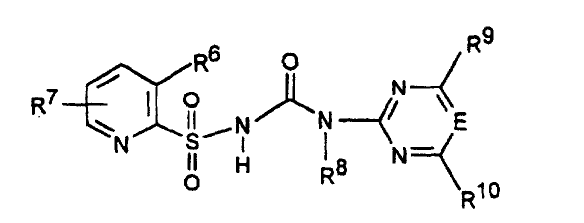 worin
      - E: CH oder N, vorzugsweise CH,
      - R⁶: lod oder NR¹¹R¹²,
      - R⁷: H, Halogen, Cyano, (C₁-C₃)-Alkyl, (C₁-C₃)-Alkoxy, (C₁-C₃)-Halogenalkyl, (C₁-C₃)-Halogenalkoxy, (C₁-C₃)-Alkylthio, (C₁-C₃)-Alkoxy- (C₁-C₃)-alkyl, (C₁-C₃)-Alkoxy-carbonyl, Mono- oder Di-((C₁-C₃)-alkyl)-amino, (C₁-C₃)-Alkylsulfinyl oder -sulfonyl, SO₂-NR^{a}R^{b} oder CO-NR^{a}R^{b}, insbesondere H,
      - R^{a}, R^{b}: unabhängig voneinander H, (C₁-C₃)-Alkyl, (C₁-C₃)-Alkenyl, (C₁-C₃)-Alkinyl oder zusammen -(CH₂)₄-, -(CH₂)₅- oder (CH₂)₂-O-(CH₂)₂-,
      - R⁸: H oder CH₃,
      - R⁹: Halogen, (C₁-C₂)-Alkyl, (C₁-C₂)-Alkoxy, (C₁-C₂)-Halogenalkyl, vorzugsweise CF₃, (C₁-C₂)-Halogenalkoxy, vorzugsweise OCHF₂ oder OCH₂CF₃,
      - R¹⁰: (C₁-C₂)-Alkyl, (C₁-C₂)-Halogenalkoxy, vorzugsweise OCHF₂, oder (C₁-C₂)-Alkoxy, und
      - R¹¹: (C₁-C₄)-Alkyl und
      - R¹²: (C₁-C₄)-Alkylsulfonyl oder
      - R¹¹ und R¹²: gemeinsam eine Kette der Formel -(CH₂)₃SO₂- oder -(CH₂)₄SO₂
      bedeuten,
      z.B. 3-(4,6-Dimethoxypyrimiden-2-yl)-1-(3-N-methylsulfonyl-N-methylaminopyridin-2-yl)-sulfonylharnstoff, oder deren Salze,
   86) Alkoxyphenoxysulfonylharnstoffe, wie sie in EP-A-0342569 beschrieben sind, vorzugsweise solche der Formel 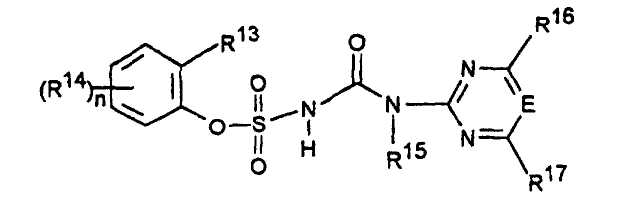 worin
      - E: CH oder N, vorzugsweise CH,
      - R¹³: Ethoxy, Propoxy oder Isopropoxy,
      - R¹⁴: Wasserstoff, Halogen, NO₂, CF₃, CN, (C₁-C₄)-Alkyl, (C₁-C₄)-Alkoxy, (C₁-C₄)-Alkylthio oder ((C₁-C₃)-Alkoxy)-carbonyl, vorzugsweise in 6-Position am Phenylring,
      - n: 1, 2 oder 3, vorzugsweise 1,
      - R¹⁵: Wasserstoff, (C₁-C₄)-Alkyl oder (C₃-C₄)-Alkenyl,
      - R¹⁶, R¹⁷: unabhängig voneinander Halogen, (C₁-C₂)-Alkyl, (C₁-C₂)-Alkoxy, (C₁-C₂)-Halogenalkyl, (C₁-C₂)-Halogenalkoxy oder (C₁-C₂)-Alkoxy-(C₁-C₂)-alkyl, vorzugsweise OCH₃ oder CH₃, bedeuten,
      z.B. 3-(4,6-Dimethoxypyrimidin-2-yl)-1-(2-ethoxyphenoxy)-sulfonylharnstoff, oder deren Salze,
   B7) Imidazolylsulfonylharnstoffe, wie
      MON 37500, Sulfosulfuron (s. Brighton Crop Prot. Conf. - Weeds - 1995, S. 57) und andere verwandte Sulfonylharnstoffderivate und Mischungen daraus.
C) Cyclohexandion-Herbizide wie
   3-(1-Allyloxyiminobutyl)-4-hydroxy-6,6-dimethyl-2-oxocyclohex-3-encarbonsäuremethylester (Alloxydim),
   2-(1-Ethoximinobutyl)-5-(2-ethylthiopropyl)-3-hydroxy-cyclohex-2-en-1-on (Sethoxydim),
   2-(1-Ethoximinobutyl)-5-(2-phenylthiopropyl)-3-hydroxy-cyclohex-2-en-1-on (Cloproxydim),
   2-(1-(3-Chlorallyloxy)iminobutyl)-5-[2-(ethylthio)propyl]-3-hydroxy-cyclohex-2-en-1-on,
   2-(1-(3-Chlorallyloxy)iminopropyl)-5-[2-(ethylthio)propyl]-3-hydroxy-cyclohex-2-en-1-on (Clethodim),
   2-(1-(Ethoxyimino)-butyl)-3-hydroxy-5-(thian-3-yl)-cyclohex-2-enon (Cycloxydim), oder
   2-(1-Ethoxyiminopropyl)-5-(2,4,6-trimethylphenyl)-3-hydroxy-cyclohex-2-en-1-on (Tralkoxydim),
D) Imidazolinon-Herbizide wie
   2-(4-lsopropyl-4-methyl-5-oxo-2-imidazolin-2-yl)-5-methylbenzoesäuremethylester und
   2-(4-lsopropyl-4-methyl-5-oxo-2-imidazolin-2-yl)-4-methylbenzoesäure (Imazamethabenz),
   5-Ethyl-2-(4-isopropyl-4-methyl-5-oxo-2-imidazolin-2-yl)-pyridin-3-carbonsäure (Imazethapyr),
   2-(4-Isopropyl-4-methyl-5-oxo-2-imidazolin-2-yl)-chinolin-3-carbonsäure (Imazaquin),
   2-(4-Isopropyl-4-methyl-5-oxo-2-imidazolin-2-yl)-pyridin-3-carbonsäure (Imazapyr), 5-Methyl-2-(4-lsopropyl-4-methyl-5-oxo-2-imidazolin-2yl)-pyridin-3-carbonsäure (Imazethamethapyr),
E) Triazolopyrimidinsulfonamidderivate wie
   N-(2,6-Difluorphenyl)-7-methyl-1,2,4-triazolo-(1,5-c)-pyrimidin-2-sulfonamid (Flumetsulam),
   N-(2,6-Dichlor-3-methylphenyl)-5,7-dimethoxy-1,2,4-triazolo-(1,5-c)-pyrimidin-2-sulfonamid,
   N-(2,6-Difluorphenyl)-7-fluor-5-methoxy-1,2,4-triazolo-(1,5-c)-pyrimidin-2-sulfonamid N-(2,6-Dichlor-3-methylphenyl)-7-chlor-5-methoxy-1,2,4-triazolo-(1,5-c)-pyrimidin-2-sulfonamid,
   N-(2-Chlor-6-methoxycarbonyl)-5,7-dimethyl-1,2,4-triazolo-(1,5-c)-pyrimidin-2-sulfonamid (siehe z. B. EP-A-343 752, US- 4 988 812),
F) Benzoylcyclohexandionderivate, z. B.
   2-(2-Chlor-4-methylsulfonylbenzoyl)-cyclohexan-1,3-dion (SC-0051, s. EP-A-137963),
   2-(2-Nitrobenzoyl)-4,4-dimethyl-cyclohexan-1,3-dion (s. EP-A-274634),
   2-(2-Nitro-3-methylsulfonylbenzoyl)-4,4-dimethyl-cyclohexan-1,3-dion (s. WO 91/13548),
G) Pyrimidinyloxy-pyrimidincarbonsäure- bzw. Pyrimidinyloxy-benzoesäure-Derivate, z.B.
   3-(4,6-Dimethoxypyrimidin-2-yl)-oxy-pyridin-2-carbonsäurebenzylester (EP-A-249 707),
   3-(4,6-Dimethoxypyrimidin-2-yl)-oxy-pyridin-2-carbonsäuremethylester (EP-A-249 707),
   2,6-Bis[(4,6-dimethoxypyrimidin-2-yl)-oxy]-benzoesäure (EP-A-321 846),
   2,6-Bis[(4,6-dimethoxypyrimidin-2-yl)-oxy]-benzoesäure-(1-ethoxycarbonyloxyethyl)-ester (EP-A-472 113) und
H) S-(N-Aryl-N-alkyl-carbamoylmethyl)-dithiophosphonsäureester wie S-[N-(4-Chlorphenyl)-N-isopropyl-carbamoylmethyl]-O,O-dimethyl-dithiophosphat (Anilofos).

Die obengenannten Herbizide der Gruppe A bis H sind dem Fachmann bekannt und in der Regel in "The Pesticide Manual",The British Crop Protection Council and the Royal Soc. of Chemistry, 10th edition, 1994 oder in "Agriculturat Chemicals Book II - Herbicides -", by W.T. Thompson, Thompson Publications, Fresno CA, USA 1990 oder in "Farm Chemicals Handbook '90", Meister Publishing Company, Willoughby OH, USA 1990 beschrieben.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und liegt vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch einfache Vorversuche ermitteln.

Haupteinsatzgebiete für die Anwendung der Safener sind vor allem Getreidekulturen (Weizen, Roggen, Gerste, Hafer, Reis, Mais, Sorghum), aber auch Baumwolle und Sojabohne, vorzugsweise Getreide, besonders bevorzugt Mais.

Ein besonderer Vorteil der erfindungsgemäßen Safener der Formel (I) ist bei deren Kombination mit Herbiziden aus der Gruppe der Sulfonylharnstoffe festzustellen. Einige Herbizide dieser Strukturklasse können speziell in Getreidekulturen z.B. Mais nicht oder nicht genügend selektiv eingesetzt werden. Durch die Kombination mit den erfindungsgemäßen Safenem sind auch bei diesen Herbiziden in Getreide oder Mais hervorragende Selektivitäten zu erreichen.

Die Safener der Formel (I) können je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandllung der angesäten, aber noch nicht bewachsenen Anbauflächen ein.

Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg, vorzugsweise 0,005 bis 0,5 kg Wirkstoff je Hektar.

Die Verbindungen der Formel (I) und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen oder Suspensionen, Suspoemulsionen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu-und Bodenapplikation, Granulate (GR) in Form von Mikro-, Sprüh-, Aufzugs- und Adsorptionsgranulaten, Granulate für die Boden- bzw. Streuapplikation, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley arid Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder ölbasis sein. Sie können beispielsweise durch Naß-Vennahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührem, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineratölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I) (Safener) oder des Safener/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

in Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weiswe verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u. a. variiert die erforderliche Aufwandmenge der Safener.

Folgende Beispiele dienen zur Erläuterung der Erfindung:

### A. Formulierungsbeispiele

a) Ein Stäubemittel wird erhalten, indem man 10 Gew.-Teile einer Verbindung der Formel (I) oder eines Wirkstoffgemischs aus einem Herbizid und einem Safener der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) oder eines Wirkstoffgemischs aus einem Herbizid und einem Safener der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) oder eines Wirkstoffgemischs aus einem Herbizid und einem Safener der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) oder eines Wirkstoffgemischs aus einem Herbizid und einem Safener der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man 75 Gewichtsteile einer Verbindung der Formel (I) oder eines Wirkstoffgemischs aus einem Herbizid und einem Safener der Formel (I),
   - 10 Gewichtsteile: ligninsulfonsaures Calcium,
   - 5 Gewichtsteile: Natriumlaurylsulfat,
   - 3 Gewichtsteile: Polyvinylalkohol und
   - 7 Gewichtsteile: Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I) oder eines Wirkstoffgemischs aus einem Herbizid und einem Safener der Formel (I),
   - 5 Gewichtsteile: 2,2'-dinaphthylmethan-6,6'-disulfonsaures Natrium
   - 2 Gewichtsteile: oleoylmethyltaurinsaures Natrium,
   - 1 Gewichtsteil: Polyvinylalkohol,
   - 17 Gewichtsteile: Calciumcarbonat und
   - 50 Gewichtsteile: Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### B. Herstellungsbeispiele

### 1. (E)-2-Fluor-3-(4-chlorphenyl)-propensäureethylester (Beispiel 1.1 aus Tabelle 1)

5,42 g (22,4 mmol) 2-Fluor-2-phosphonoessigsäuretriethylester werden in 35 ml THF bei -78 °C unter Argon vorgelegt und mit 8,92 ml einer 2,5 m BuLi-Lösung (22,4 mmol) versetzt. Nach 30 min bei dieser Temperatur werden 2,81 g (20 mmol) 4-Chlorbenzaldehyd - in 50 ml THF gelöst - zugetropft. Nach 2 h bei- 78 °C wird weitere 5 h bei Raumtemperatur nachgerührt. Nach Zugabe von ca. 60 ml halbkonz. HCI wird die organische Phase abgetrennt, mit 2 x 25 ml ges. NaCl-Lösung sowie 25 ml Wasser gewaschen, getrocknet und eingeengt, wobei das Produkt als farbloses Öl erhalten wird.
Ausbeute: 2,59 g (57 %), ¹H-NMR (CDCl₃, ppm; TMS): d= 1,25 (t, 3H), 4,25 (q, 2H), 6,82 (d, 24 Hz, 1H), 7,35 (dd, 4H)

### 2. (E)-2-Fluor-3-(4-trifluormethylphenyl)-propensäureethylester (Beispiel 1.2 aus Tabelle 1)

5,33 g (22 mmol) 2-Fluor-2-phosphonoessigsäuretriethylester werden in 35 ml THF bei -78 °C unter Argon vorgelegt und mit 10 ml einer 2,5 m BuLi-Lösung (26 mmol) versetzt. Nach 30 min bei dieser Temperatur werden 5,33 g (22 mmol) 4-Trifluormethylbenzaldehyd - in 50 ml THF gelöst - zugetropft. Nach 2 h bei- 78 °C wird weitere 5 h bei Raumtemperatur nachgerührt. Nach Zugabe von ca. 60 ml halbkonz. HCI wird die organische Phase abgetrennt, mit 2 x 25 ml ges. NaCI-Lösung sowie 25 ml Wasser gewaschen, getrocknet und eingeengt, wobei das Produkt als Harz erhalten wird.
Ausbeute: 4,12 g (71 %), ¹H-NMR (d₆-DMSO, ppm; TMS): d= 1,17 (t, 3H), 4,20 (q, 2H), 7,31 (d, 24 Hz, 1H), 7,72 (dd, 4H)

### 3. (Z)-2-Fluor-3-(4-chlorphenyl)-propensäureethylester (Beispiel 2.1 aus Tabelle 2)

6 g (26,3 mmol) Natrium-diethyl-oxalofluoracetat werden bei Raumtemperatur in 200 ml THF suspendiert und mit 3,7 g (26,3 mmol) 4-Chlorbenzaldehyd versetzt. Nach 3-stündigem Erhitzen unter Rückfluß wird die Mischung eingeengt, in Diethylether aufgenommen und mit ges. KHCO₃-Lösung verrührt. Nach Abtrennung der organischen Phase wird mit MgSO₄ getrocknet und die Lösung eingeengt. Nach Kugelrohrdestillation erhält man das Produkt als erstarrtes Harz.
Ausbeute: 4,5 g (75 %), ¹H-NMR (d₆-DMSO, ppm; TMS): d=1,31 (t, 3H), 4,32 (q, 2H), 7,10 (d, 36 Hz, 1H), 7,62 (dd, 4H)

### 4. (Z)-2-Fluor-3-(4-trifluormethylphenyl)-propensäureethylester (Beispiel 2.2 aus Tabelle 2)

26,2 g (115 mmol) Natrium-diethyl-oxalofluoracetat werden bei Raumtemperatur in 120 ml THF suspendiert und mit 20 g (115 mmol) 4-Trifluormethylbenzaldehyd versetzt. Nach 3stdg. Erhitzen auf Rückfluß wird die Mischung eingeengt, in Diethylether aufgenommen und mit ges. KHCO₃-Lösung verrührt. Nach Abtrennung der organischen Phase wird mit MgSO₄ getrocknet und die Lösung eingeengt. Nach Kugelrohrdestillation erhält man das Produkt als erstarrtes Harz.
Ausbeute: 21,4 g (71 %), ¹H-NMR (d₆-DMSO, ppm; TMS): d=1,35 (t, 3H), 4,32 (q, 2H), 7,20 (d, 35 Hz, 1H), 7,85 (dd, 4H)

### 5. (Z)-2-Fluor-3-(4-trifluormethylphenyl)-propensäure (Beispiel 2.32 aus Tabelle 2)

15,0 g (57 mmol) (Z)-Ethyl-2-fluor-3-(4-trifluormethylphenyl)-propenoat werden bei Raumtemperatur in 150 ml Methanol suspendiert und mit 21.0 g (0.52 mol) Natriumhydroxid - in 50 ml Wasser gelöst - versetzt. Nach 1stdg. Rühren bei Raumtemperatur wird die Mischung eingeengt und mit 2n HCI auf pH 4 eingestellt. Der ausfallende Niederschlag wird abgesaugt, mit Wasser gewaschen und getrocknet.
Ausbeute: 10,8 g (81 %), Smp.: 210 °C

### 6. (Z)-2-Fluor-3-(4-chlorphenyl)-propensäure (Beispiel 2.49 aus Tabelle 2)

12,8 g (56 mmol) (Z)-Ethyl-2-fluor-3-(4-chlorphenyl)-propenoat werden bei Raumtemperatur in 100 ml Methanol suspendiert und mit 21.0 g (0.52 mol) Natriumhydroxid - in 50 ml Wasser gelöst - versetzt. Nach 1stdg. Rühren bei Raumtemperatur wird die Mischung eingeengt und mit 2n HCI auf pH 4 eingestellt. Der ausfallende Niederschlag wird abgesaugt, mit Wasser gewaschen und getrocknet.
Ausbeute: 11,5 g (92 %), Smp.: 285 °C

### 7. (Z)-Butyl-2-fluor-3-(4-trifluormethylphenyl)-propenoat (Beispiel 2.34 aus Tabelle 2)

5,0 g (19 mmol) (Z)-Ethyl-2-fluor-3-(4-trifluormethylphenyl)-propenoat werden bei Raumtemperatur in 40 ml Butanol vorgelegt, mit 1 ml Titantetraisopropylat versetzt und 4 h unter Rückfluß erhitzt. Nach Einengen der Reaktionsmischung i. Vak. bis zur Trockne wird das Rohprodukt durch Säulenchromatographie gereinigt (Laufmittel: Petrolether/Essigsäureethylester = 9/1).
Ausbeute: 4,9 g (89 %), ¹H-NMR (CDCl₃, ppm, TMS): 0.98 (t, 3H), 1,44 (m, 2H), 1,73 (m, 2H), 4,29 (t, 2H), 6,86 (d, 34 Hz, 1H), 7,38 (dd, 2H), 7,58 (d, 2H)

In den nachfolgenden Tabellen sind beispielhaft eine Reihe von Verbindungen der allgemeinen Formel (I) aufgeführt, die in analoger Weise erhalten werden können:

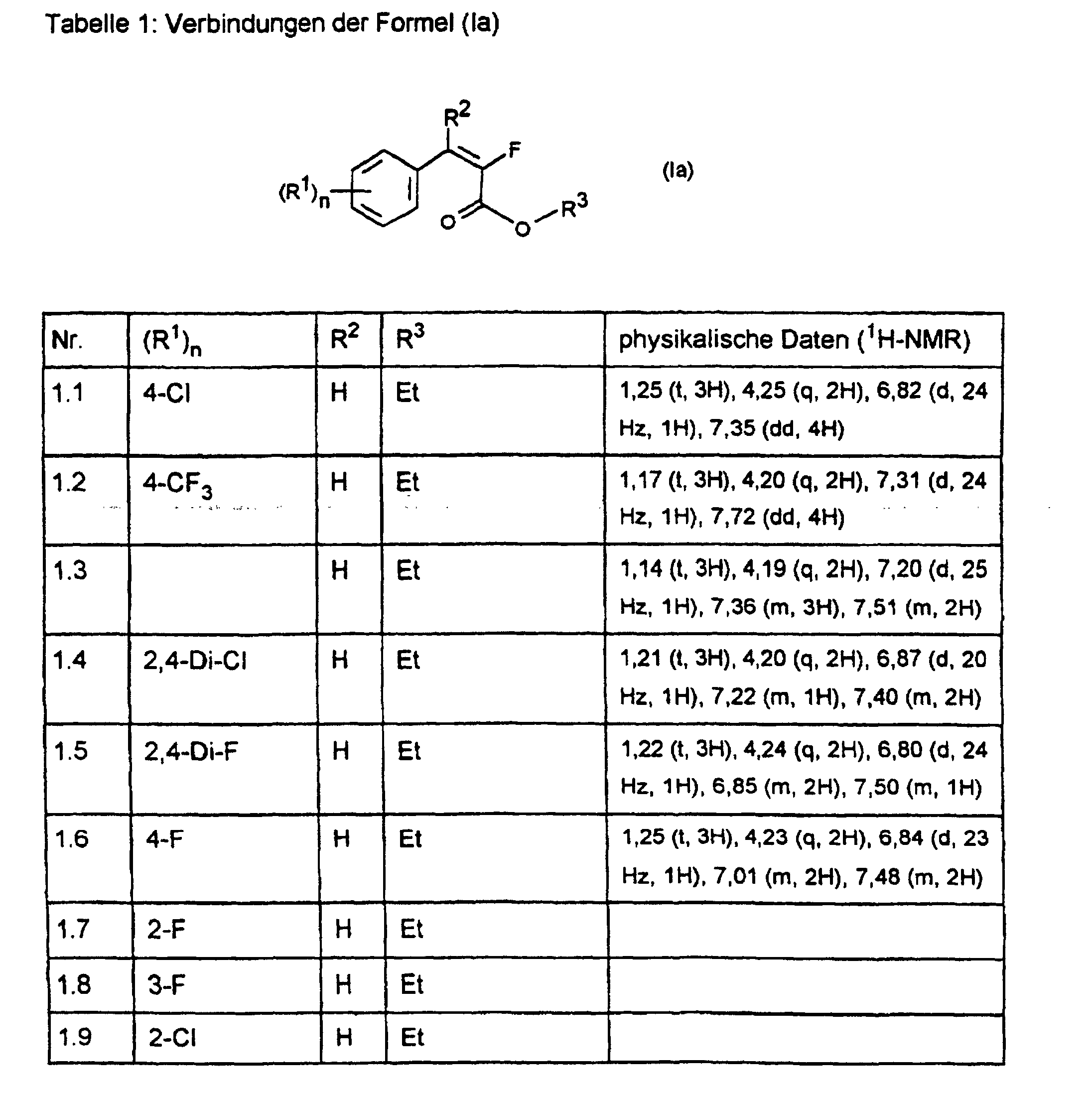

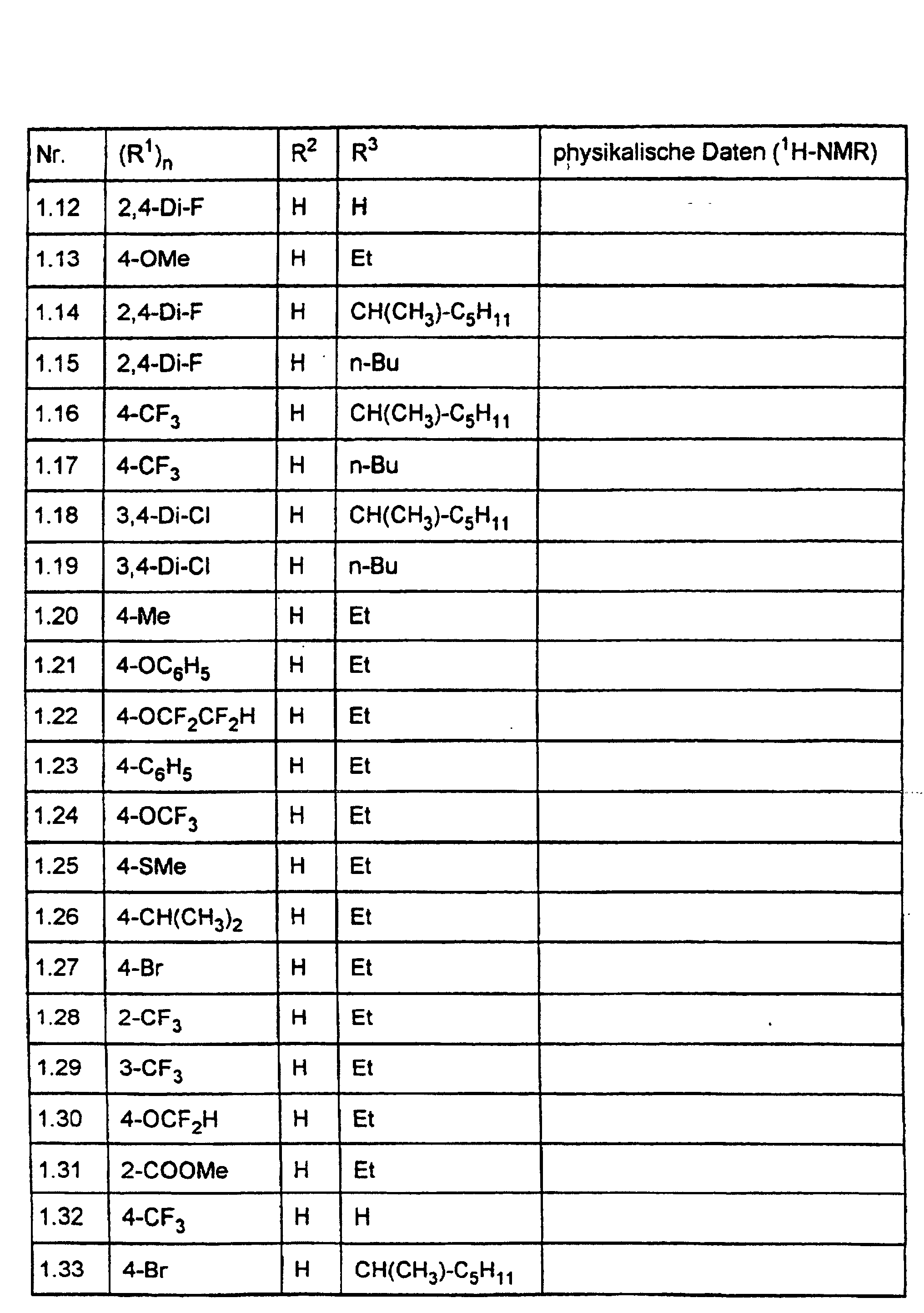

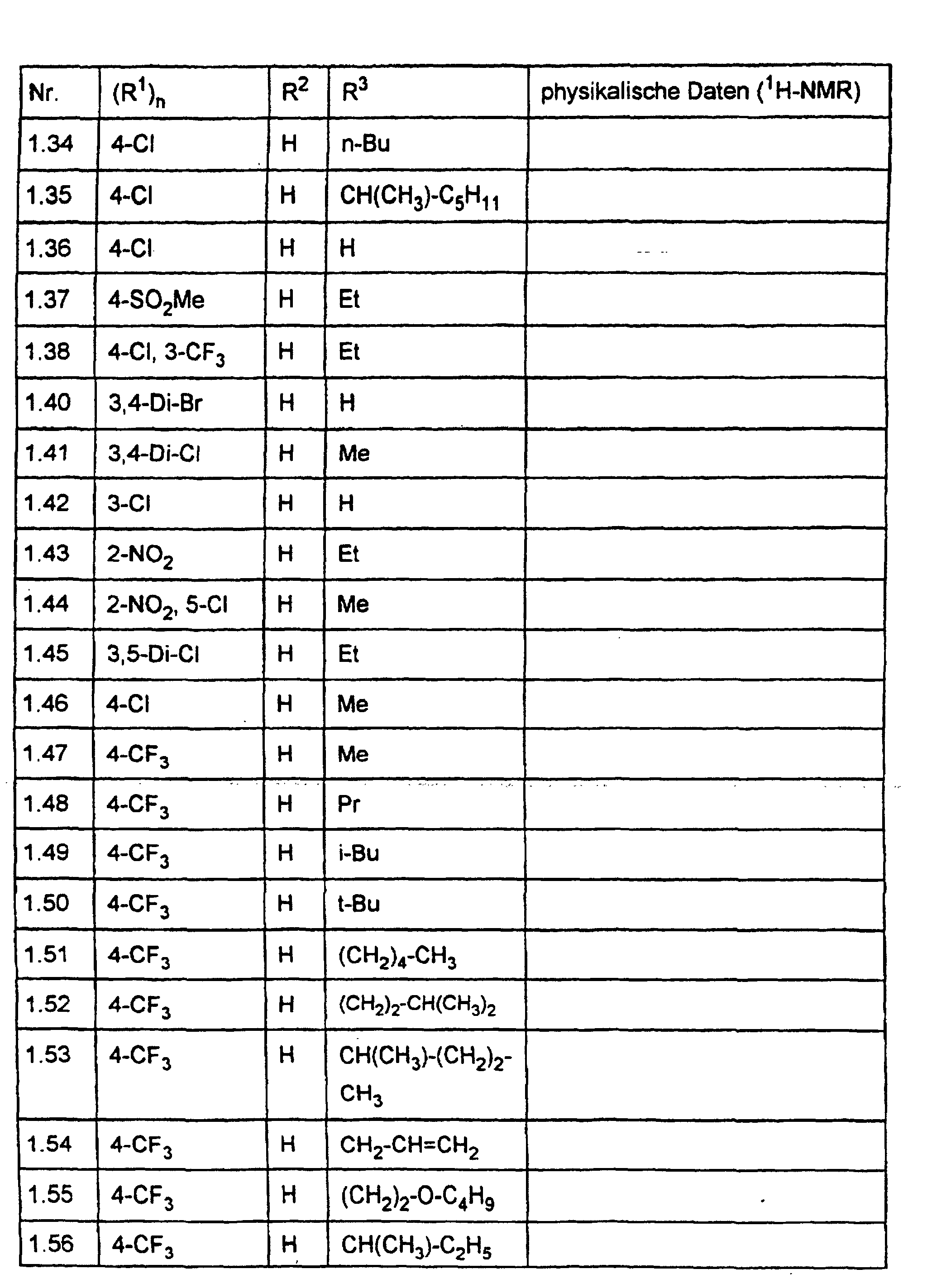

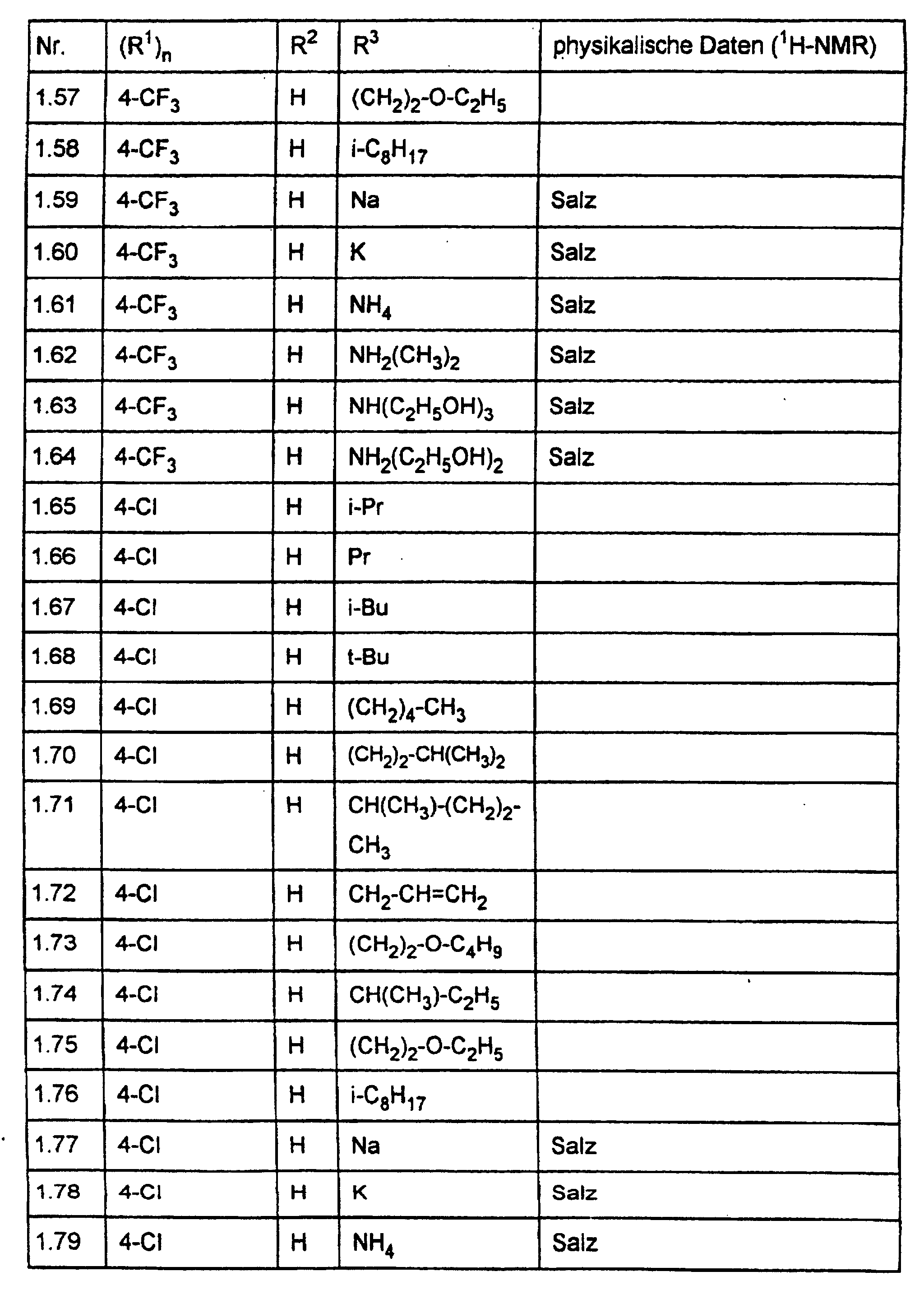

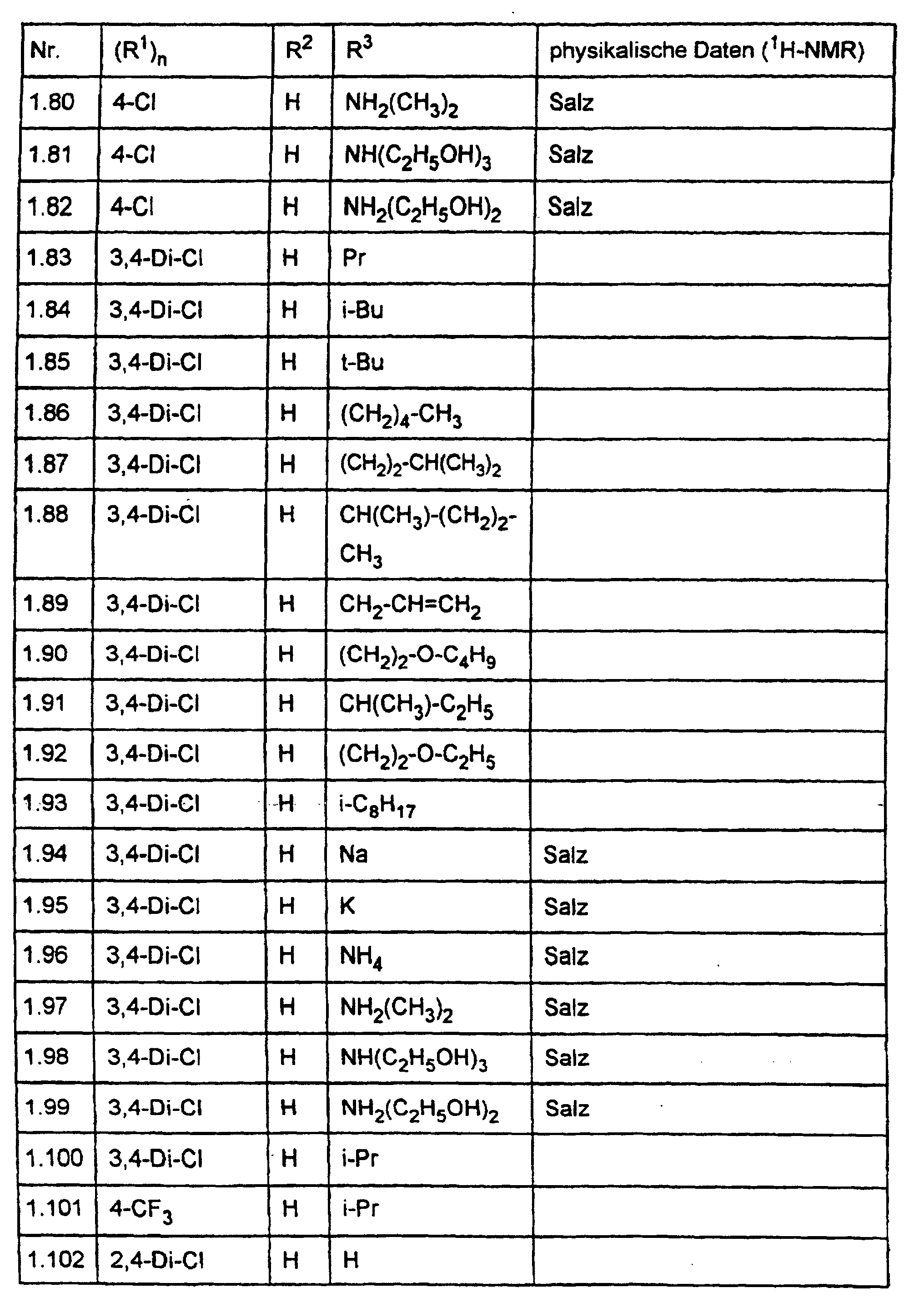

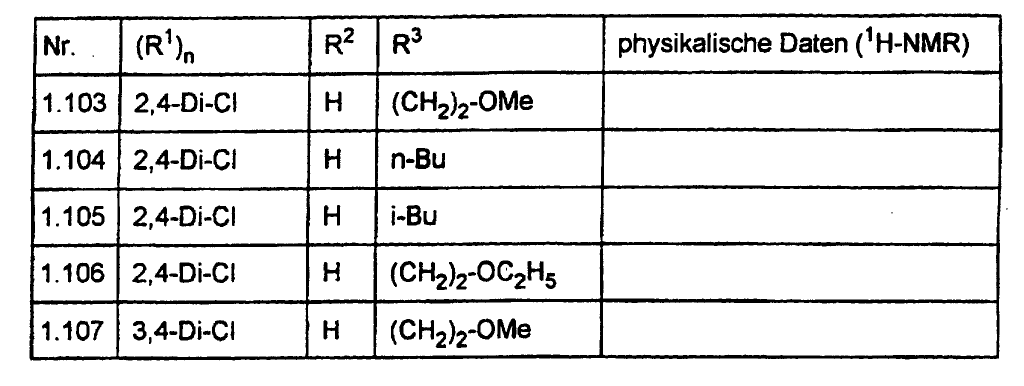

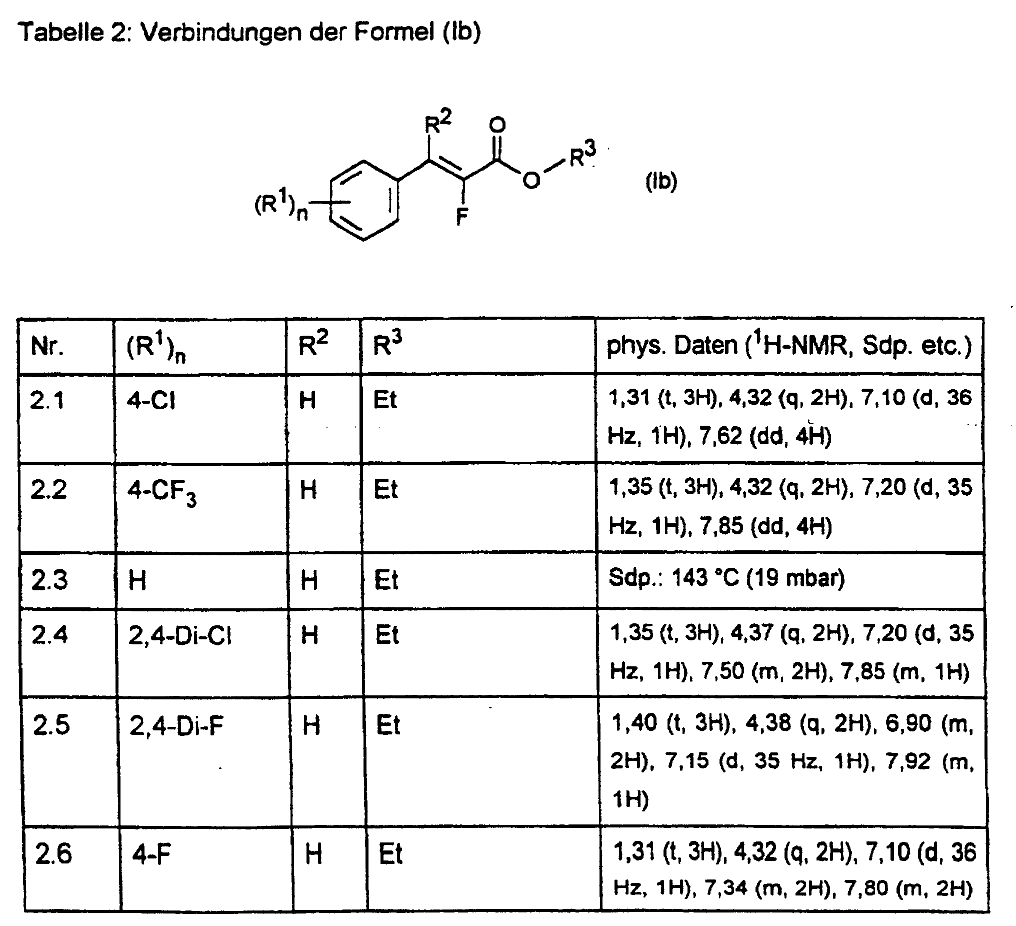

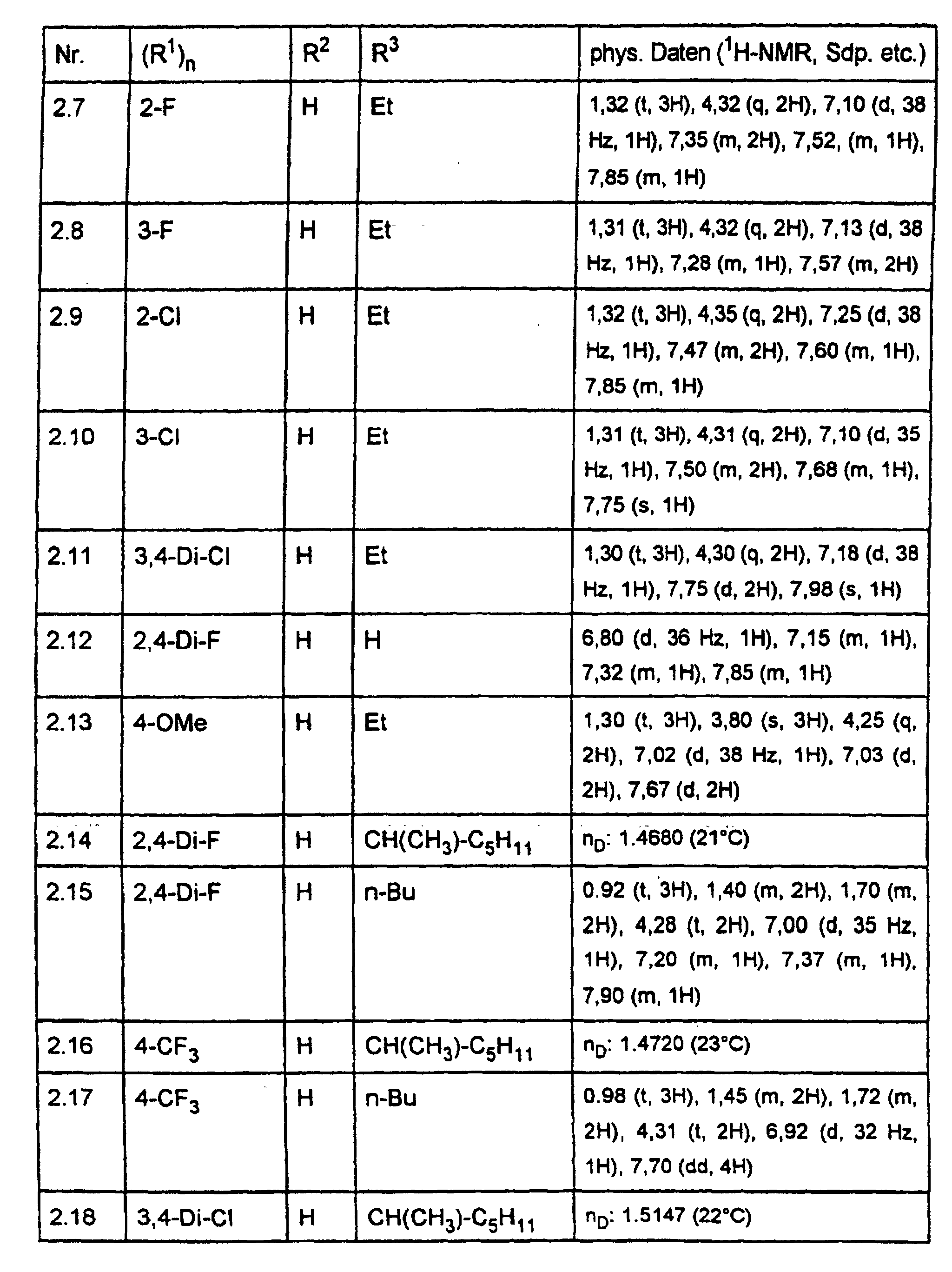

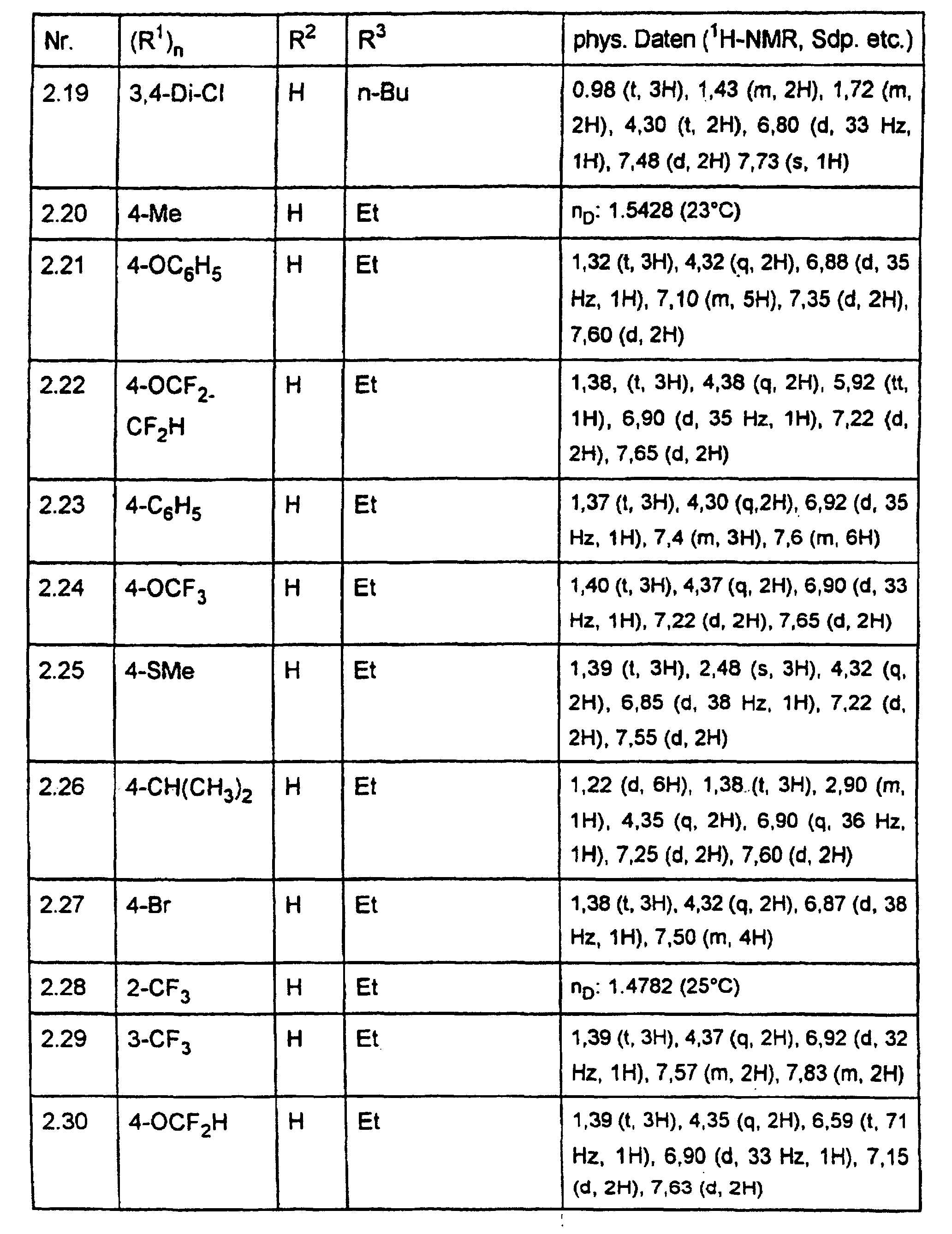

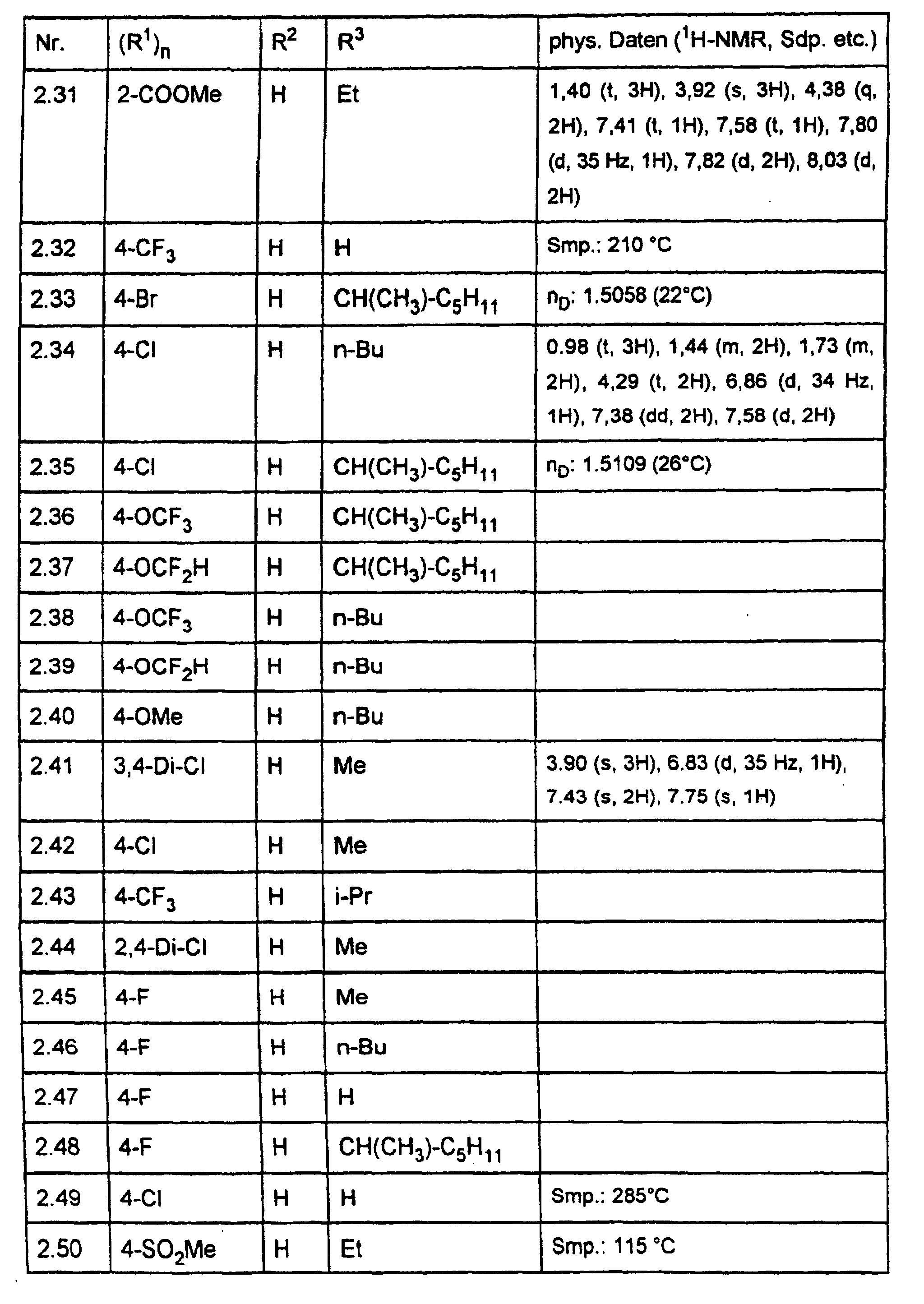

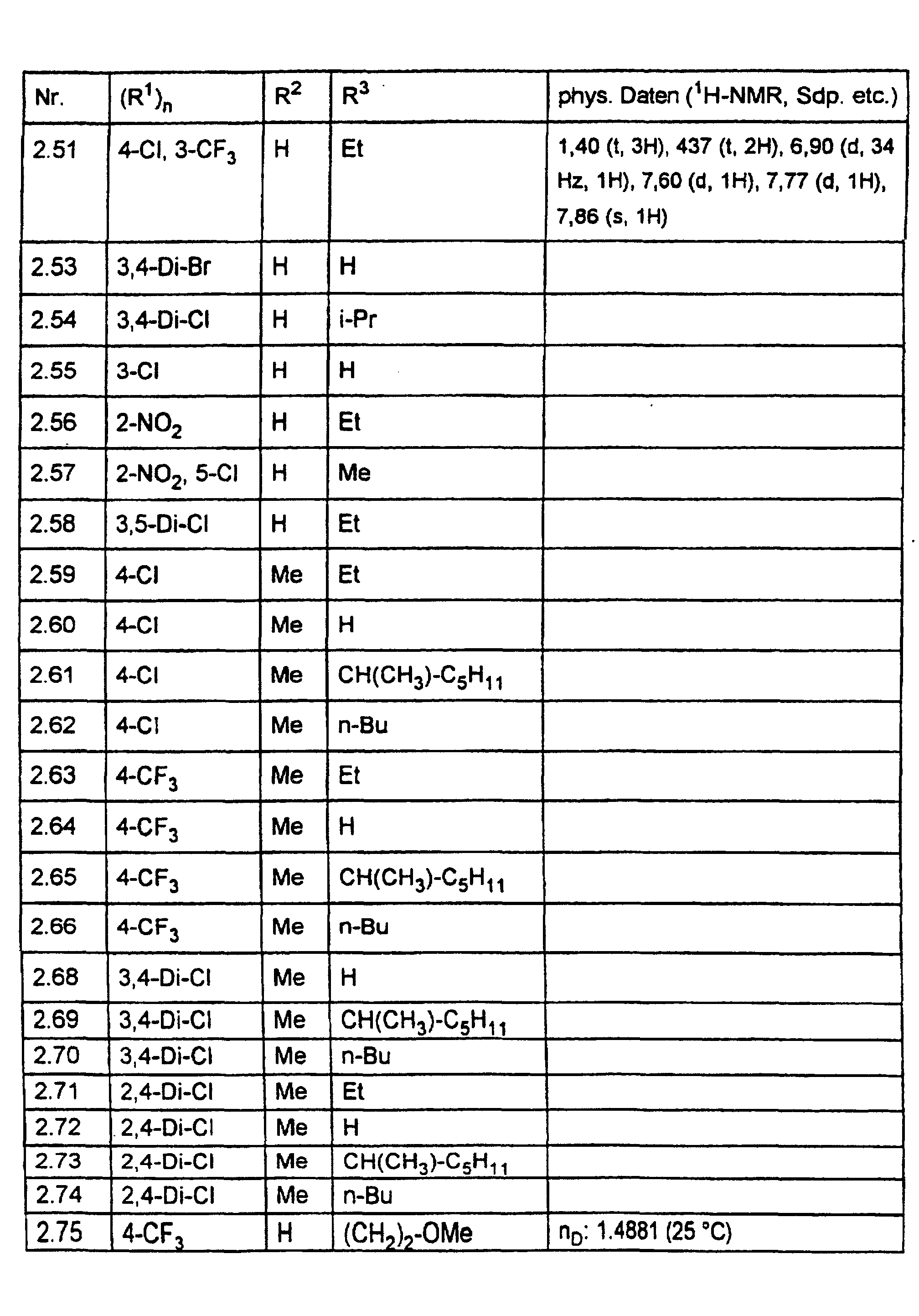

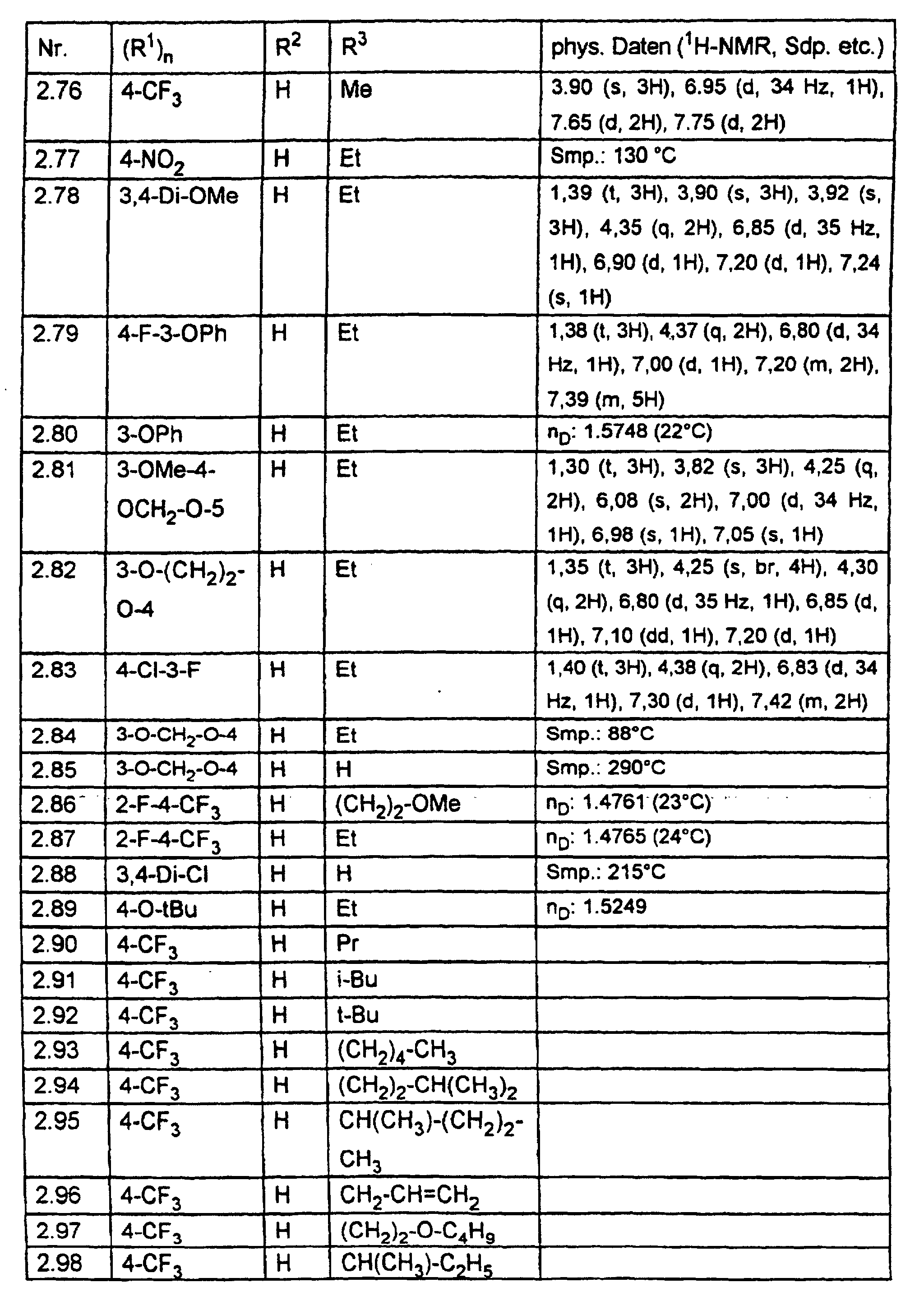

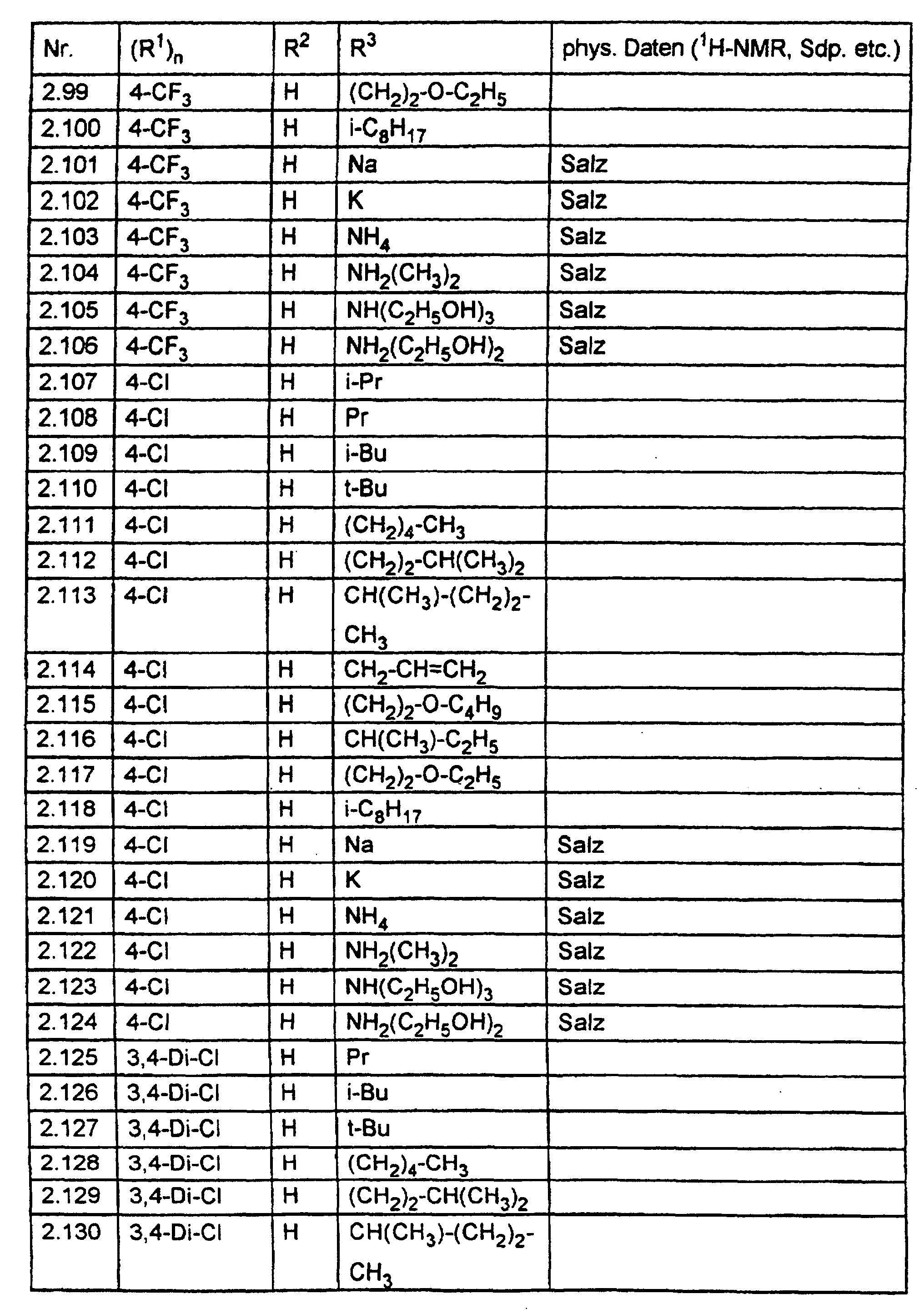

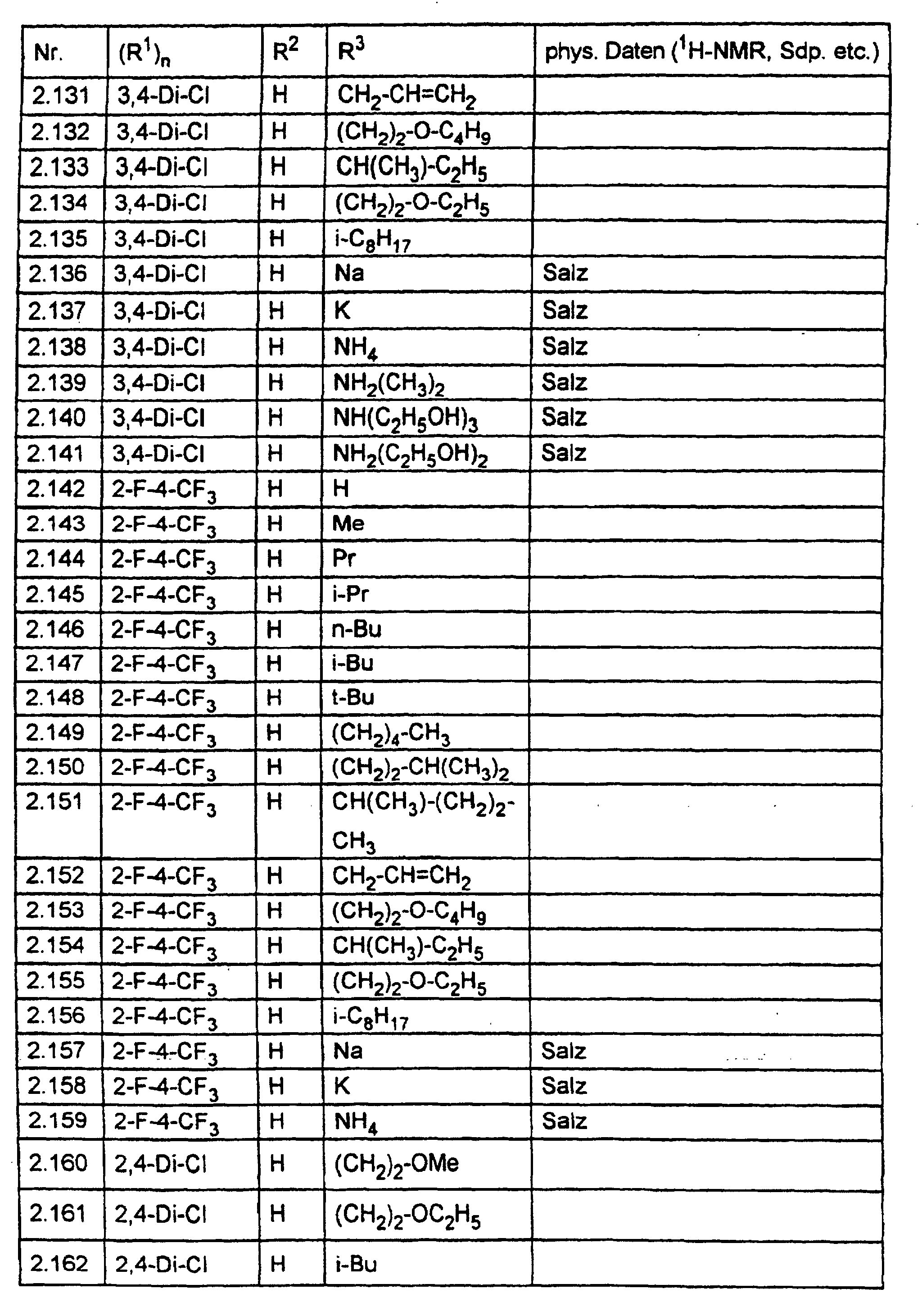

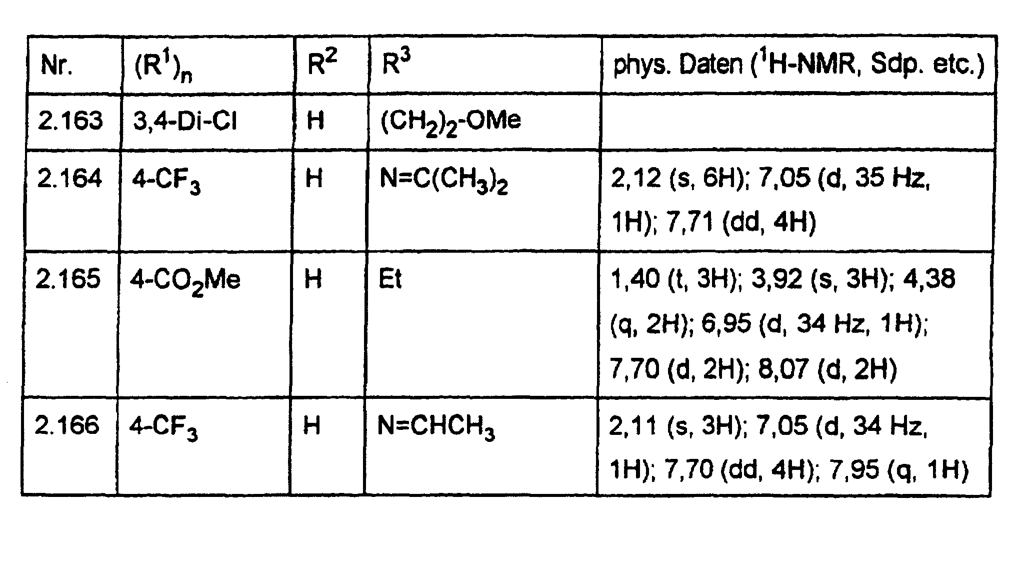

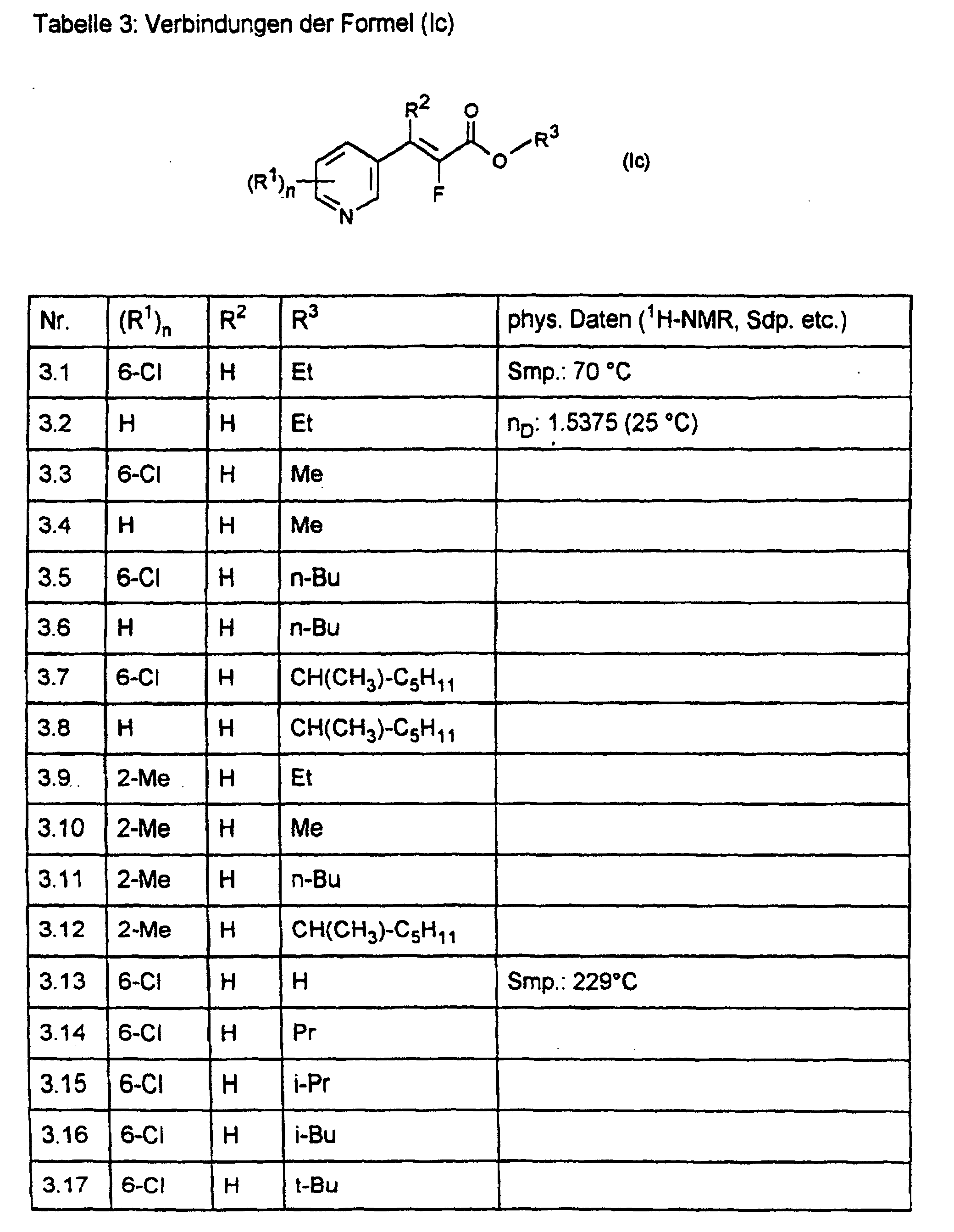

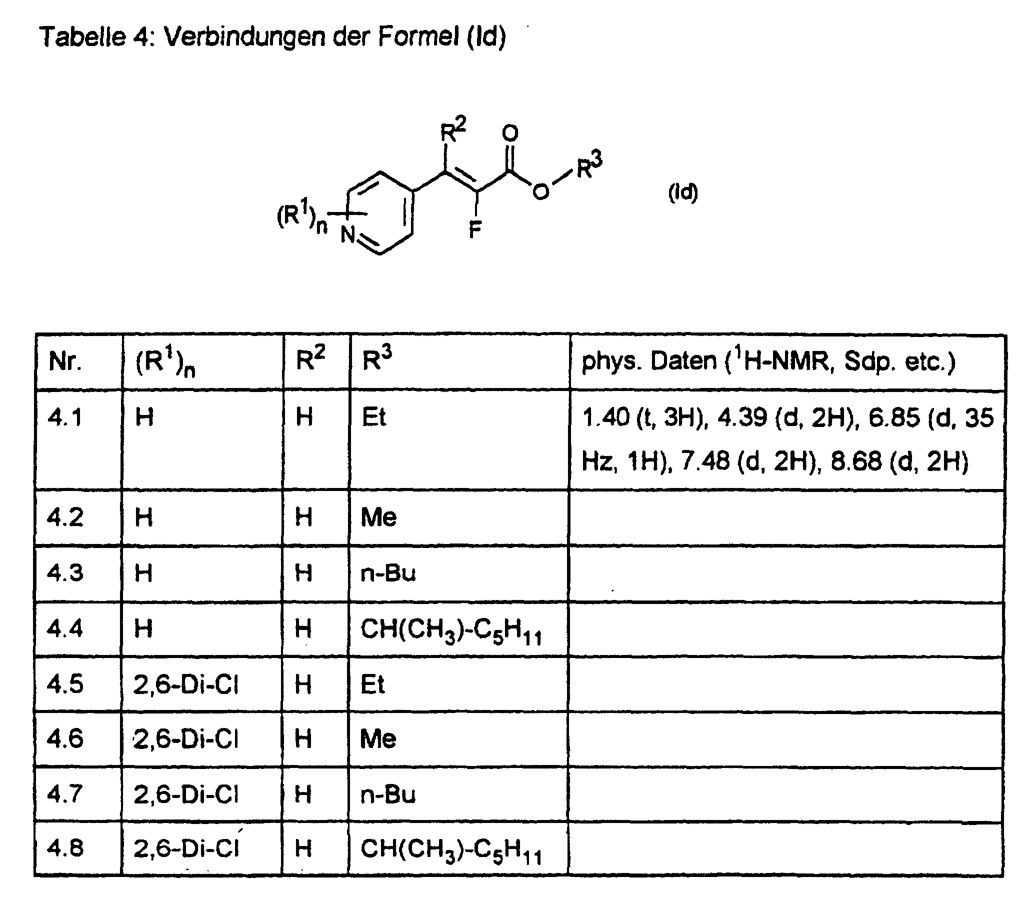

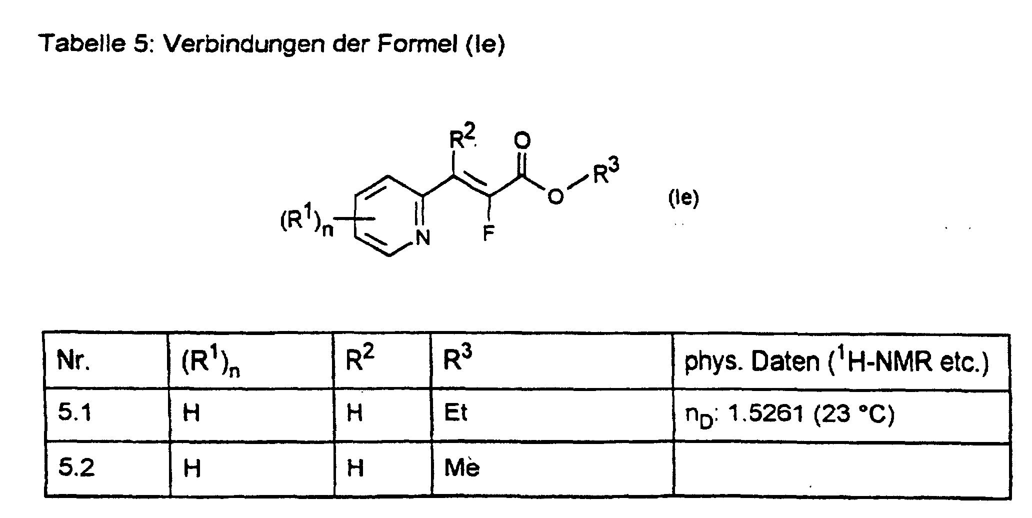

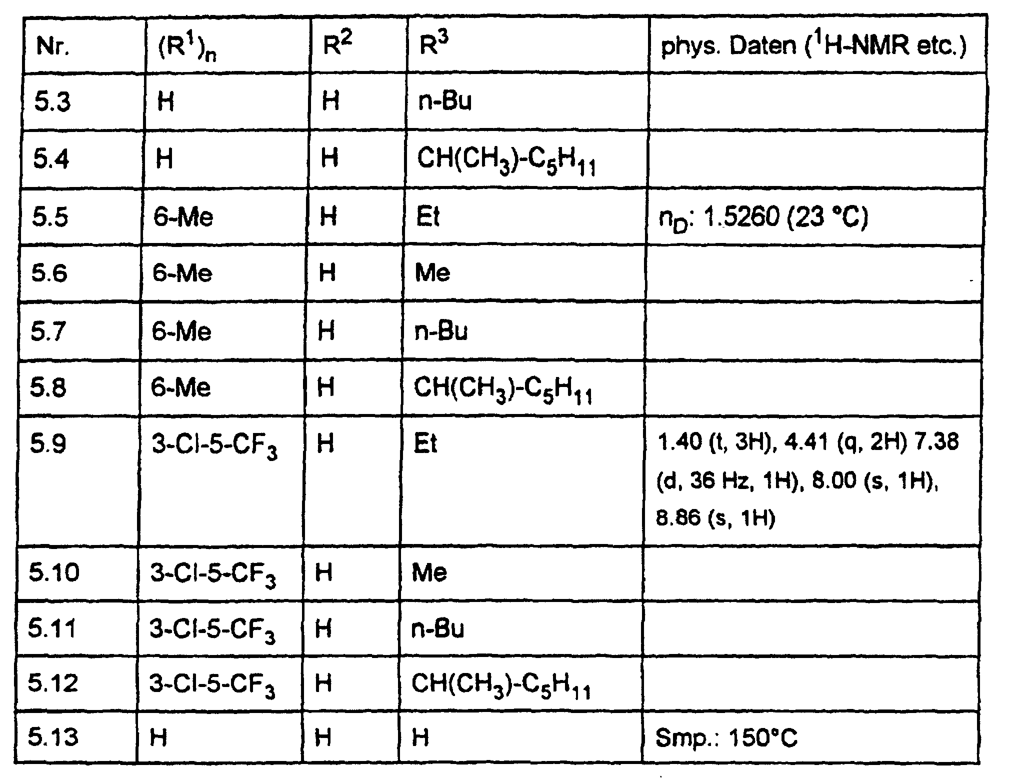

Abkürzungen in den Tabellen 1-5:
Me = CH₃, Et = C₂H₅, Pr = C₃H₇ = n-Propyl, i-Pr = Isopropyl, Bu = C₄H₉ = n-Butyl,
i-Bu = Isobutyl, t-Bu = tertiär-Butyl,
Sdp. = Siedepunkt, Smp. = Schmelzpunkt, n_{D} = Brechungsindex angegeben als Refraktometerwert der Natrium-D-Linie.

Wenn (R¹)ₙ = H, dann entspricht dies dem unsubstituierten Fall (n=0).

### C. Biologische Beispiele

Samen von Gerste, Reis oder Mais wurden in sandiger Lehmerde in Plastiktöpfen ausgelegt, im Gewächshaus bis zum 3- bis 4- Blattstadium herangezogen und nacheinander mit den erfindungsgemäßen Verbindungen und den Herbiziden im Nachlaufverfahren behandelt. Die Herbizide und die Verbindungen der Formel (I) wurden dabei in Form wäßriger Suspensionen bzw. Emulsionen mit einer Wasseraufwandmenge von umgerechnet 300 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen.
Einige Versuchsergebnisse sind in den Tabellen 6, 7 und 8 zusammengestellt:

**Tabelle 6:**

| Safenerwirkung in Gerste | | |
|---|---|---|
| Produkt Herbizid/Safener | Dosis (kg a.i./ha) | herbizide Wirkung in % HORVU |
| H₁ | 0,2 | 80 |
| H₁ + Nr. 2.41 | 0,2 + 1,25 | 35 |
| H₁ + Nr. 2.3 | 0,2 + 1,25 | 55 |
| H₁ + Nr. 2.5 | 0,2 + 1,25 | 60 |
| H₁ + Nr. 2.6 | 0,2 + 1,25 | 45 |
| H₁ + Nr. 1.2 | 0,2 + 1,25 | 60 |
| H₁ + Nr. 2.1 | 0,2 + 1,25 | 30 |
| H₁ + Nr. 2.4 | 0,2 + 1,25 | 20 |
| H₁ + Nr. 2.11 | 0,2 + 1,25 | 25 |

H₁ = Fenoxaprop-ethyl
HORVU = Hordeum vulgare (Gerste)
Nr. ... = Safener von Beispiel Nr.... aus Abschnitt B (Chemische Beispiele)

**Tabelle 7:**

| Safenerwirkung in Reis | | |
|---|---|---|
| Produkt Herbizid/Safener | Dosis (kg a.i./ha) | herbizide Wirkung in % ORYSA |
| H₁ | 0,3 | 85 |
| H₁ + Nr. 2.41 | 0,3 + 1,25 | 65 |
| H₁ + Nr. 2.56 | 0,3 + 1,25 | 65 |
| H₁ + Nr. 2.27 | 0,3 + 1,25 | 65 |

H₁ = Fenoxaprop-ethyl
ORYSA = Oryza sativa (Reis)
Nr. ... = Safener von Beispiel Nr. ... aus Abschnitt B (Chemische Beispiele)

**Tabelle 8:**

| Safenerwirkung in Mais | | |
|---|---|---|
| Produkt Herbizid/Safener | Dosis (kg a.i./ha) | herbizide Wirkung in % ZEAMA |
| H₂ | 0,075 | 90 |
| H₂ + Nr. 2.41 | 0,075 + 1,25 | 50 |
| H₂ + Nr. 2.27 | 0,075 + 1,25 | 65 |
| H₂ + Nr. 1.2 | 0,075 + 1,25 | 45 |
| H₂ + Nr. 2.1 | 0,075 + 1,25 | 60 |
| H₂ + Nr. 2.2 | 0,075 + 1,25 | 40 |
| H₂ + Nr. 2.17 | 0,075 + 1,25 | 45 |
| H₂ + Nr. 2.16 | 0,075 + 1,25 | 40 |

H₂ = 3-(4,6-Dimethoxypyrimidin-2-yl)-1-[3-(N-methyl-N-methylsulfonyl-amino)-2-pyridylsulfonyl]-harnstoff
ZEAMA = Zea mays (Mais)
Nr.... = Safener von Beispiel Nr.... aus Abschnitt B (Chemische Beispiele)

## Patentansprüche

1. Herbizid-Safener-Kombination, enthaltend
A) mindestens einen herbiziden Wirkstoff
sowie
B) mindestens eine Verbindung der Formel (I) 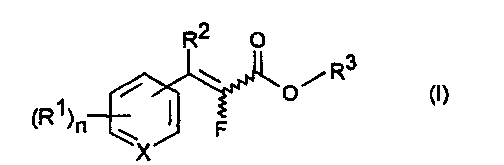
in welcher
X CH oder N bedeutet;
n für den Fall, daß X = N ist, eine ganze Zahl von 0 bis 4 und für den Fall, daß X = CH ist, eine ganze Zahl von 0 bis 5 bedeutet;
R¹ Halogen, (C₁-C₄)-Alkyl, (C₁-C₄)-Halogenalkyl, (C₁-C₄)-Alkoxy, (C₁-C₄)-Halogenalkoxy, Nitro, (C₁-C₄)-Alkylthio, (C₁-C₄)-Alkylsulfonyl, (C₁-C₄)-Alkoxycarbonyl, Phenyl oder Phenoxy bedeutet, wobei die beiden letztgenannten Reste unsubstituiert oder durch einen oder mehrere, vorzugsweise bis zu drei gleiche oder verschiedene Reste aus der Gruppe, bestehend aus Halogen, (C₁-C₄)-Alkyl, (C₁-C₄)-Halogenalkyl, (C₁-C₈)-Alkoxy, Halogen-(C₁-C₈)-alkoxy, Nitro, Amino, Mono- oder Di-(C₁-C₄)-alkylamino und Cyano,substituiert ist, oder auch für den Fall, daß n eine ganze Zahl größer 1 ist, zwei der Reste R¹ gemeinsam ein unsubstituiertes oder substituiertes 1,ω-Dioxoalkylen bedeuten;
R² Wasserstoff oder (C₁-C₄)-Alkyl bedeutet und
R³ Wasserstoff, (C₁-C₈)-Alkyl,(C₂-C₄)-Alkenyl oder (C₂-C₄)-Alkinyl bedeutet, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und (C₁-C₄)-Alkoxy substituiert ist,
bedeuten.

2. Herbizid-Safener-Kombination nach Anspruch 1, enthaltend
A) mindestens einen herbiziden Wirkstoff ausgewählt aus der Gruppe, bestehend aus Phenoxyphenoxycarbonsäureestern, Heteroaryloxycarbonsäureestem, Sulfonylharnstoffen, Cyclohexandionen, Benzoylcyclohexandionen, Imidazolinonen, Triazolopyrimidinsulfonamiden, Pyrimidinyloxy-pyrimidincarbonsäurederivaten, Pyrimidinyloxy-benzoesäurederivaten und S-(N-Aryl-N-alkylcarbamoylmethyl)-dithiophosphonsäureestem.

3. Herbizid-Safener-Kombination nach Anspruch 1 oder 2, **dadurch gekennzeichnet, daß** das Mischungsverhältnis von Komponente B zu Komponente A in einem Bereich zwischen 1:10 und 10:1 liegt.

4. Pflanzenschutzmittel enthaltend eine Herbizid-Safener-Kombination nach einem der Ansprüche 1 bis 3.

5. Pflanzenschutzmittel nach Anspruch 4, **dadurch gekennzeichnet, daß** eine Herbizid-Safener-Kombination nach einem der Ansprüche 1 bis 5 zu 0,1 bis 99 Gew.-% neben den im Pflanzenschutz üblichen Formulierungsmitteln enthalten ist.

6. Verfahren zum Schutz von Kulturpflanzen vor phytotoxischen Nebenwirkungen von Herbiziden, **dadurch gekennzeichnet, daß** eine wirksame Menge mindestens einer Verbindung der Formel (I) vor, nach oder gleichzeitig mit einem herbiziden Wirkstoff auf Pflanzen, Pflanzensamen oder die Anbaufläche appliziert wird, wobei die Verbindung der Formel (I) oder deren Salz sowie das Herbizid gemäß einem der Ansprüche 1 oder 2 definiert sind.

7. Verwendung von 2-Fluoracrylsäurederivaten der Formel (I) zum Schutz von Kulturpflanzen vor phytotoxischen Nebenwirkungen von Herbiziden, wobei die Verbindung der Formel (I) oder deren Salz sowie das Herbizid gemäß einem der Ansprüche 1 bis 2 definiert sind.

8. 2-Fluoracrylsäurederivate der Formel (I) 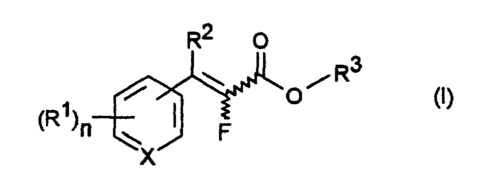 in welcher
X, n, R¹, R² und R³ wie in Anspruch 1 definiert sind,
ohne dabei
a) α-Fluorzimtsäure oder deren Methyl- oder Ethylester,
b) 3- oder 4-Methyl-α-fluorzimtsäureethylester,
c) 3- oder 4-Chlor-α-fluorzimtsäure oder deren Methyl- oder Ethylester,
d) 2-Hydroxy-α-fluorzimtsäureethylester,
e) α-Fluor-α-2-pyridylacrylsäureethylester,
f) α-Fluor-α-3-pyridylacrylsäureethylester,
g) 3- oder 4-Methoxy-α-fluorzimtsäureethylester,
h) 3- oder 4-Phenoxy-α-fluorzimtsäureethylester,
j) 4-Phenyl-α-fluorzimtsäureethylester,
k) 3- oder 4-Fluor-α-fluorzimtsäure oder deren Methyl- oder Ethylester,
l) 3- oder 4-Brom-α-fluorzimtsäure oder deren Ethylester,
m) 4-Carboxyethyl-α-fluorzimtsäureethylester,
n) 3- oder 4-Trifluormethyl-α-fluorzimtsäuremethyl- oder ethylester,
o) 3- oder 4-Cyano-α-fluorzimtsäureethylester,
p) 3- oder 4-Nitro-α-fluorzimtsäureethylester,
q) 3,4-Dichlor-α-fluorzimtsäureethylester,
r) α-Fluor-β-methylzimtsäure oder deren Ethylester,
s) 4-Methyl-α-fluorzimtsäure-t-butylester,
t) 3,4-Dibrom-α-fluorzimtsäureethylester,
u) β-Ethyl-α-fluor-zimtsäureethylester zu bedeuten.

9. Verfahren zur Herstellung von 2-Fluoracrylsäurederivaten gemäß Anspruch 8, **dadurch gekennzeichnet, daß** man einen Aldehyd oder ein Keton der Formel (II), 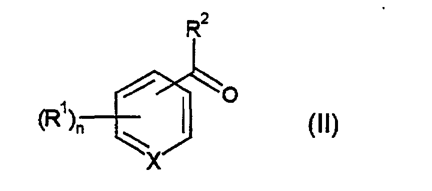 worin
X, R¹, R² und n wie in Formel (I) definiert sind,
a) mit Diethyloxalofluoroacetat oder
b) mit 2-Fluor-2-phosphonoessigsäuretriethylester
zum Ethylester umsetzt, und diesen, falls R³ ungleich Ethyl ist, anschließend nach üblichen Verfahren zur Verbindung der Formel (I) umsetzt.

## Claims

1. A herbicide/safener combination, which comprises
A) at least one herbicidally active compound
and
B) at least one compound of the formula (I) 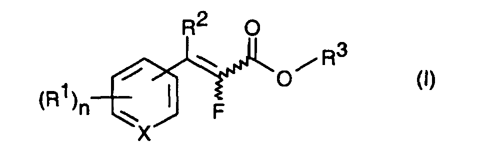 in which
X is CH or N;
n, in the case that X = N, is an integer from 0 to 4 and,
in the case that X = CH, is an integer from 0 to 5;
R¹ is halogen, (C₁-C₄)-alkyl, (C₁-C₄)-haloalkyl, (C₁-C₄)-alkoxy, (C₁-C₄)-haloalkoxy, nitro, (C₁-C₄)-alkylthio, (C₁-C₄)-alkylsulfonyl, (C₁-C₄)-alkoxycarbonyl, phenyl or phenoxy, where the last two radicals are unsubstituted or substituted by one or more, preferably up to three, identical or different radicals selected from the group consisting of halogen, (C₁-C₄)-alkyl, (C₁-C₄)-haloalkyl, (C₁-C₈)-alkoxy, halo-(C₁-C₈)-alkoxy, nitro, amino, mono- or di-(C₁-C₄)-alkylamino and cyano, or two of the radicals R¹ together, in the case that n is an integer greater than 1, are an unsubstituted or substituted 1,ω-dioxoalkylene;
R² is hydrogen or (C₁-C₄)-alkyl and
R³ is hydrogen, (C₁-C₈)-alkyl, (C₂-C₄)-alkenyl or (C₂-C₄)-alkynyl, where each of the abovementioned carbon-containing radicals is unsubstituted or substituted by one or more, preferably up to three, identical or different radicals selected from the group consisting of halogen and (C₁-C₄)-alkoxy.

2. The herbicide/safener combination as claimed in claim 1, which comprises
A) at least one herbicidally active compound selected from the group consisting of phenoxyphenoxycarboxylic esters, heteroaryloxycarboxylic esters, sulfonylureas, cyclohexanediones, benzoylcyclohexanediones, imidazolinones, triazolopyrimidinesulfonamides, pyrimidinyloxypyrimidinecarboxylic acid derivatives, pyrimidinyloxybenzoic acid derivatives and S-(N-aryl-N-alkylcarbamoylmethyl)dithiophosphonic esters.

3. The herbicide/safener combination as claimed in claim 1 or 2, wherein the mixing ratio of component B to component A is in the range between 1:10 and 10:1.

4. A crop protection agent which comprises a herbicide/safener combination as claimed in any of claims 1 to 3.

5. The crop protection agent as claimed in claim 4, which comprises 0.1 to 99% by weight of a herbicide/safener combination as claimed in any of claims 1 to 3 in addition to formulating agents customarily used in crop protection.

6. A method for protecting crop plants against phytotoxic side effects of herbicides, which comprises applying an effective amount of at least one compound of the formula (I) before, after or together with a herbicidally active compound to plants, seeds of plants or the area under cultivation, the compound of the formula (I) or the salt thereof and the herbicide being defined as claimed in either of claims 1 and 2.

7. The use of 2-fluoroacrylic acid derivatives of the formula (I) for protecting crop plants against phytotoxic side effects of herbicides, the compound of the formula (I) or the salt thereof and the herbicide being defined as claimed in either of claims 1 and 2.

8. A 2-fluoroacrylic acid derivative of the formula (I) 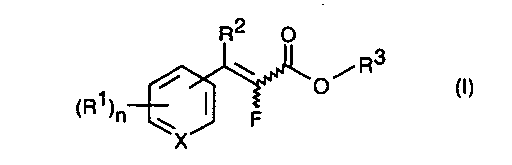 in which
X, n, R¹, R² and R³ are each as defined in claim 1,
except for
a) α-fluorocinnamic acid or the methyl or ethyl ester thereof,
b) ethyl 3- or 4-methyl-α-fluorocinnamate,
c) 3- or 4-chloro-α-fluorocinnamic acid or the methyl or ethyl ester thereof,
d) ethyl 2-hydroxy-α-fluorocinnamate,
e) ethyl α-fluoro-α-2-pyridylacrylate,
f) ethyl-α-fluoro-α-3-pyridylacrylate,
g) ethyl 3- or 4-methoxy-α-fluorocinnamate,
h) ethyl 3- or 4-phenoxy-α-fluorocinnamate,
j) ethyl 4-phenyl-α fluorocinnamate,
k) 3- or 4-fluoro-α-fluorocinnamic acid or the methyl or ethyl ester thereof,
l) 3- or 4-bromo-α-fluorocinnamic acid or the ethyl ester thereof,
m) ethyl 4-carboxyethyl-α-fluorocinnamate,
n) methyl or ethyl 3- or 4-trifluoromethyl-α-fluorocinnamate,
o) ethyl 3- or 4-cyano-α-fluorocinnamate,
p) ethyl 3- or 4-nitro-α-fluorocinnamate,
q) ethyl 3,4-dichloro-α-fluorocinnamate,
r) α-fluoro-β-methylcinnamic acid or the ethyl ester thereof,
s) t-butyl 4-methyl-α-fluorocinnamate,
t) ethyl 3,4-dibromo-α-fluorocinnamate,
u) ethyl β-ethyl-α-fluorocinnamate.

9. A process for preparing 2-fluoroacrylic acid derivatives as claimed in claim 8, which comprises reacting an aldehyde or a ketone of the formula (II) 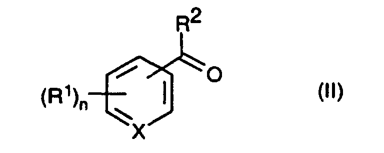 in which
X, R¹, R² and n are each as defined in formula (I)
a) with diethyloxalofluoroacetate or
b) with triethyl 2-fluoro-2-phosphonoacetate
to give the ethyl ester which is, if R³ is not ethyl, subsequently reacted by customary methods to give the compound of the formula (I).

## Revendications

1. Combinaison herbicide-antidote renfermant
A) au moins une substance active herbicide
ainsi que
B) au moins un composé de formule (I) 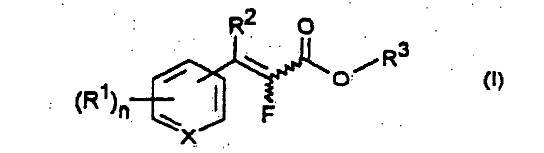
dans laquelle
X représente des groupes CH ou N ;
n dans le cas où X = N, est un entier de 0 à 4 et dans le cas où X = CH, est un entier de 0 à 5 ;
R¹ représente des groupes halogène, alkyle en C₁-C₄, halogénoalkyle en C₁-C₄, alkoxy en C₁-C₄, halògénoalkoxy en C₁-C₄, nitro, (alkyl en C₁-C₄)-thio, (alkyl en C₁-C₄)sulfonyle, (alkoxy en C₁-C₄)-carbonyle, phényle ou phénoxy, les deux derniers restes sont non substitués ou substitués par un ou plusieurs, de préférence par jusqu'à trois restes identiques ou différents pris dans le groupe comprenant halogène, alkyle en C₁-C₄, halogénoalkyle en C₁-C₄, alkoxy en C₁-C₈, halogénoalkoxy en C₁-C₈, nitro, amino, mono- ou di-(alkyl en C₁-C₄)amino et cyano, ou aussi dans le cas où n est un entier supérieur à 1, deux des restes R¹ représentent conjointement un groupé 1,ω-dioxoalkylène non substitué ou substitué ;
R² représente un atome d'hydrogène ou un groupe alkyle en C₁-C₄ et
R³ représente un atome d'hydrogène, des groupes alkyle en C₁-C₈, alcényle en C₂-C₄ ou alcynyle en C₂-C₄, chacun des restes carbonés précités étant non substitué ou substitué par un ou plusieurs, de préférence par jusqu'à trois restes identiques ou différents pris dans le groupe comprenant halogène et alkoxy en C₁-C₄,
ou leurs sels.

2. Combinaison herbicide-antidote selon la revendication 1 renfermant
A) au moins une substance active herbicide prise dans le groupe comprenant des esters d'acides phénoxyphénoxycarboxyliques, des esters d'acides hétéroarylcarboxyliques, des sulfonylurées, des cyclohexanediones, des benzoylcyclohexanediones, des imidazolinones, des triazolopyrimidinesulfonamides, des dérivés d'acides pyrimidinyloxy-pyrimidine-carboxyliques, des dérivés d'acides pyrimidinyloxy-benzoïques et des S-(N-aryl-N-alkyl-carbamoylméthyl)-dithiophosphonates.

3. Combinaison herbicide-antidote selon la revendication 1 ou 2, **caractérisée en ce que** le rapport de mélange du composant B au composant A est compris entre 1:10 et 10:1.

4. Agent phytosanitaire renfermant une combinaison herbicide-antidote selon l'une des revendications 1 à 3.

5. Agent phytosanitaire selon la revendication 4, **caractérisé** qu'il renferme une combinaison herbicide-antidote selon l'une des revendications 1 à 5 dans une quantité de 0,1 à 99 % en poids, au côté des agents de formulation usuels dans le domaine phytosanitaire.

6. Procédé pour la protection des plantes de culture contre les effets secondaires phytotoxiques des herbicides, **caractérisé en ce qu'**on applique une quantité efficace d'au moins un composé de formule (I) avant, après ou en même temps qu'une substance active herbicide aux plantes, aux grains de plantes ou à la surface cultivable, le composé de formule (I) ou leurs sels ainsi que l'herbicide sont définis selon l'une des revendications 1 ou 2.

7. Utilisation de dérivés de l'acide 2-fluoroacrylique de formule (I) pour la protection de plantes de culture contre les effets secondaires phytotoxiques des herbicides, le composé de formule (I) ou son sel ainsi que l'herbicide étant définis selon une ou plusieurs des revendications 1 ou 2.

8. Dérivés de l'acide 2-fluoracrylique de formule (I) 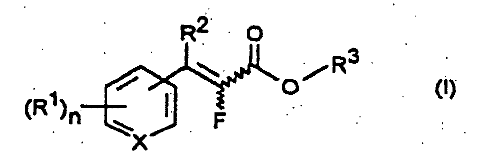 dans laquelle
X, n, R¹, R² et R³ sont définis comme à la revendication 1,
sans qu'ils représentent un des composés suivants
a) acide α-fluorocinnamique ou ses esters méthylique ou éthylique,
b) 3- ou 4-méthyl-α-fluorocinnamate d'éthyle,
c) acides 3- ou 4-chloro-α-fluorocinnamiques ou leurs esters méthylique ou éthylique,
d) 2-hydroxy-α-fluorocinnamate d'éthyle,
e) α-fluoro-α-2-pyridylacrylate d'éthyle,
f) α-fluoro-α-3-pyridylacrylate d'éthyle,
g) 3- ou 4-méthoxy-α-fluorocinnamate d'éthyle,
h) 3- ou 4-phénoxy-α-fluorocinnamate d'éthyle,
j) 4-phényl-α-fluorocinnamate d'éthyle,
k) acides 3- ou 4-fluoro-α-fluorocinnamiques ou leurs esters méthylique ou éthylique,
l) acides 3- ou 4-bromo-α-fluorocinnamiques ou leur ester éthylique,
m) 4-carboxyéthyl-α-fluorocinnamate d'éthyle,
n) 3- ou 4-trifluorométhyl-α-fluorocinnamate de méthyle ou d'éthyle,
o) 3- ou 4-cyano-α-fluorocinnamate d'éthyle,
p) 3- ou 4-nitro-α-fluorocinnamate d'éthyle,
q) 3,4-dichloro-α-fluorocinnamate d'éthyle,
r) acide α-fluoro-β-méthylcinnamate et son ester d'éthyle,
s) 4-méthyl-α-fluorocinnamate de t-butyle,
t) 3,4-dibromo-α-fluorocinnamate d'éthyle,
u) β-éthyl-α-fluorocinnamate d'éthyle.

9. Procédé pour la préparation de dérivés de l'acide 2-fluoroacrylique selon la revendication 8, **caractérisé en ce qu'**on fait réagir un aldéhyde ou une cétone de formule (II) 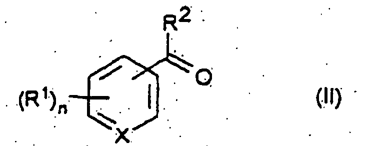 où
X, R¹, R² et n sont définis comme à la formule (I), sur
a) l'oxalofluoroacétate de diéthyle ou
b) le 2-fluoro-2-phosphonoacétate de triéthyle
pour obtenir l'ester éthylique et ensuite faire réagir celui-ci, dans le cas où R³ ne représente pas un groupe éthyle, selon des procédés usuels pour obtenir le composé de formule (I).
